# EUROPÄISCHE PATENTSCHRIFT

(11) **EP 1 599 493 B1**
(45) Veröffentlichungstag und Bekanntmachung des Hinweises auf die Patenterteilung: **24.11.2010**
(21) Anmeldenummer: 04712548.9
(22) Anmeldetag: 19.02.2004
(51) Int. Cl.: C07J 41/00, A61K 31/565, A61P 35/00

(54) **ANTITUMOR WIRKSAME 2-SUBSTITUIERTE ESTRA-1,3,5(10)-TRIEN-3-YL SULFAMATE**
2-SUBSTITUTED ESTRA-1,3,5(10)-TRIENE-3-YL SULFAMATE WITH AN ANTI-TUMOUR ACTION
SULFAMATE ESTRA-1,3,5(10)-TRIENE-3-YLE 2-SUBSTITUE A EFFET ANTITUMEUR

(30) Priorität: 19.02.2003 DE 10307104
(43) Veröffentlichungstag der Anmeldung: 30.11.2005
(73) Patentinhaber: Sterix Limited, Slough, Berkshire SL1 3XE (GB)
(72) Erfinder: HILLISCH, Alexander, 42553 Velbert, (DE); PETERS, Olaf, 99891 Tabarz (DE); GEGE, Christian, 89584 Ehingen/Donau (DE); REGENHARDT, Wilko, 37520 Osterode (DE); KOSEMUND, Dirk, 99091 Erfurt (DE); SIEMEISTER, Gerhard, 13503 Berlin (DE); UNGER, Eberhard, 07751 Cospeda (DE); BOTHE, Ulrich, 10551 Berlin (DE)
(74) Vertreter: Alcock, David
(86) Internationale Anmeldenummer: PCT/EP2004/001606
(87) Internationale Veröffentlichungsnummer: WO 2004/074307

(56) Entgegenhaltungen:
- WO-A-01/18028
- WO-A-99/33858
- MACCARTHY-MORROGH L ET AL: "DIFFERENTIAL EFFECTS OF ESTRONE AND ESTRONE-3-O-SULFAMATE DERIVATIVES ON MITOTIC ARREST, APOPTOSIS AND MICROTUBULE ASSEMBLY IN HUMAN BREAST CANCER CELLS" CANCER RESEARCH, AMERICAN ASSOCIATION FOR CANCER RESEARCH, BALTIMORE, MD, US, Bd. 60, Nr. 19, 1. Oktober 2000 (2000-10-01), Seiten 5441-5450, XP001031282 ISSN: 0008-5472 in der Anmeldung erwähnt
- PUROHIT A ET AL: "RECENT ADVANCES IN THE DEVELOPMENT OF STEROID SULPHATASE INHIBITORS" JOURNAL OF STEROID BIOCHEMISTRY AND MOLECULAR BIOLOGY, ELSEVIER SCIENCE LTD., OXFORD, GB, Bd. 69, Nr. 1/6, 1999, Seiten 227-238, XP000852540 ISSN: 0960-0760 in der Anmeldung erwähnt
- SINGH A ET AL.: "Inhibition of deoxyglucose uptake in MCF-7 breast cancer cells by 2-methoxyestrone and 2-methoxyestrone-3-O-sulfamate" MOLECULAR AND CELLULAR ENDOCRINOLOGY, Bd. 160, 2000, Seiten 61-66, XP002281331 in der Anmeldung erwähnt

## Beschreibung

Die vorliegende Erfindung betrifft 2-substituierte Estra-1,3,5(10)-trien-3-yl sulfamate und deren Verwendung zur Herstellung von Arzneimitteln, die eine Antitumor wirksame Aktivität aufweisen.

Mikrotubuli sind Organellen, die in den meisten eukaryontischen Zellen vorkommen und dort eine Reihe von Funktionen wie Mitose, intrazelluläre Bewegungen, Zellwanderung und die Ausprägung der Zellform übernehmen. Mikrotubuli sind Polymere aus Tubulin, das seinerseits ein Dimer aus einer α- und einer β-Einheit darstellt. Diese Heterodimere binden zwei Moleküle Guanosintriphosphat (GTP), wobei eines der GTPs fest gebunden und das andere austauschbar ist. Die Heterodimere polymerisieren in einer Kopf-Schwanz-Anordnung zu fadenförmigen Makromolekülen, den sogenannten Protofilamenten, die sich ihrerseits zu röhrenförmigen Organellen, den Mikrotubuli, zusammenlagern. Mikrotubuli unterliegen einem ständigen Auf- und Abbau. Das Gleichgewicht zwischen Wachstum und Abbau hängt von der Verfügbarkeit neuer GTP-Tubulin-Untereinheiten und der Hydrolysegeschwindigkeit des zweiten gebundenen GTPs ab. Am Plus-Ende werden neue Untereinheiten angebaut, wogegen am Minus-Ende Untereinheiten abdiffundieren. Es ist bekannt, dass zytotoxische Substanzen wie Colchicin, Vinblastin, Vincristin, Taxol, Epothilone, Podophyllotoxin, Steganicin, Combretastatin, 2-Methoxyestradiol den Auf- bzw. Abbau der Mikrotubuli (Tubulinpolymerisation und Tubulindepolymerisation) beeinflussen und somit in der Lage sind, die Zellteilung phasenspezifisch zu beeinflussen. Dies betrifft vor allem schnell wachsende, neoplastische Zellen, deren Wachstum durch intrazelluläre Regelmechanismen weitgehend unbeeinflusst ist. Wirkstoffe dieser Art sind prinzipiell geeignet zur Behandlung maligner Tumoren.

Fotsis et. al. Nature 1994 368, 237-239 berichten darüber, dass 2-Methoxyestradiol das Tumorwachstum und die Angiogenese hemmt.

Cushman et. al. J. Med. Chem. 1995 38, 2041-2049 untersuchen die zytotoxische sowie die Tubulin-Polymerisationshemmende Wirkung von 2-Methoxyestradiol, und berichten in J. Med. Chem. 1997, 40, 2323-2334 darüber, dass 2-Alkoxy-6-oximinoestradiol-Derivate die Tubulinpolymerisation sowie die Bindung von [³H]-Cholchicin an Tubulin hemmen. Die hier genannten 2-Alkoxy-6-oximinoestradiol-Derivate zeigen bzgl. der Hemmung der Tubulinpolymerisation vergleichbare Aktivität wie 2-Ethoxyestradiol, das eine höhere Aktivität als 2-Methoxyestradiol aufweist.

Steroid-3-sulfamate werden andererseits in der Literatur als Hemmstoffe der Steroidsulfatase beschrieben:

WO93/05064 betrifft u.a. Verbindungen der Formel 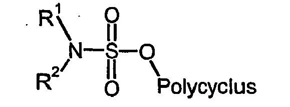 wobei R¹ und R² jeweils unabhängig Wasserstoff oder Methylgruppe bedeuten, mit der Maßgabe, dass mindestens einer der Reste R¹ und R² ein H-Atom ist, und der Rest-O-Polycyclus ein 3-Sterol ist, dessen Sulfatester durch ein Enzym mit Steroidsulfatase-Aktivität hydrolisierbar ist. In der 2-Stellung des Steroidgerüstes spezifisch substituierte Verbindungen sind nicht explizit offenbart.

US 6,011,024 beruht auf der WO93/05064 und deckt z. B. alle Verbindungen ab, in denen die primäre Sulfamatfunktion an einem Sechsring gebunden ist. In der 2-Stellung des Steroidgerüstes spezifisch substituierte Verbindungen sind wiederum nicht explizit offenbart.

WO 96/05216 betrifft an C2-unsubstituierte Estra-1,3,5(10)-trien-Sulfamat-Derivate.

WO 96/05217 betrifft pharmazeutische Zusammensetzungen enthaltend Wirkstoffe der allgemeinen Formel 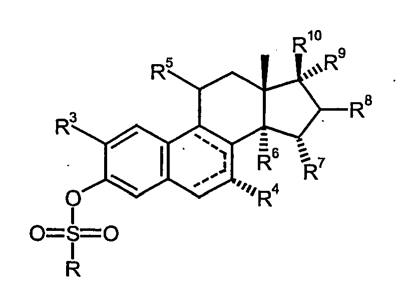 worin R = NH₂; R³ = C₁₋₅-Alkoxygruppe, OH; R⁸, R⁹ und R¹⁰ voneinander unabhängig = H, OH; R⁹ und R¹⁰ zusammen = O die Bedeutung haben können. Die darin offenbarten pharmazeutischen Zusammensetzungen können zur weiblichen Fertilitätskontrolle; klimakterischen HRT und zur Behandlung gynäkologischer und andrologischer Krankheitsbilder, wie Mamma-, bzw. Prostatakarzinom verwendet werden.

WO 97/14712 betrifft Steroidsulfamat-Derivate der allgemeinen Formel 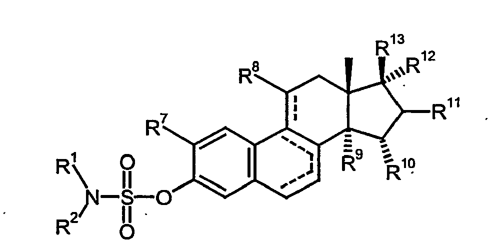 worin R¹ eine Acyl-, Alkoxycarbonyl-, Aminocarbonyl-, Sulfonyl- oder Sulfonamidylgruppe; R² ein Wasserstoff- oder ein Metallatom; R⁷ und R⁸ unabhängig voneinander H, OH und C₁₋₅-Alkoxy; R¹³, R¹², R¹¹ unabhängig voneinander H oder OH darstellen können.

WO 98/42729 betrifft 16-Halogen-substituierte Estra-1,3,5(10)-trien-3-monosulfamate sowie 3,17β-Bissulfamate, die an C2 alkoxysubstituiert sein können. Die 16-Halogensubstitution erhöht sowohl die Sulfatase-Hemmwirkung als auch die Estrogenität der entsprechenden Sulfamat-Derivate.

Die Einführung einer zu der 3-Sulfamat- zusätzlichen 17-Sulfamatfunktion setzt die Estrogenität drastisch herab.

WO 98/24802 betrifft Sulfamate, die die Estronsulfatase hemmen. 2-Methoxyestronsulfamat wird explizit genannt. Als potentielles Therapiegebiet findet

Mammakarzinom, nicht jedoch Prostatakarzinom in der Beschreibung Erwähnung. Auch WO 99/33858 beschreibt Estronsulfatase-Inhibitoren der Formel 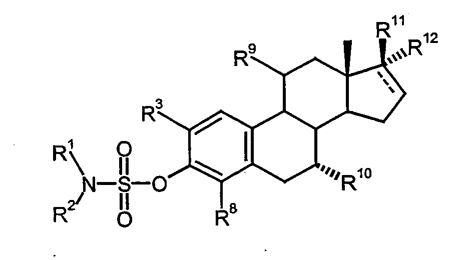 worin R¹ und R² unabhängig voneinander H, Alkyl, oder zusammen Piperidin-, Morpholin, Piperazin; R³ = H, CN, NO₂, CO₂R⁴; R^{8.} = H, NO₂, NR⁶R⁷ darstellen. In der Beschreibung ist als mögliches Therapiegebiet Mammakarzinom erwähnt.

WO 99/64013 betrifft eine pharmazeutische Zusammensetzung eines Sulfamat-Derivates mit einem Zellsignalmodifier (wie z.B. TNFα). 2-Methoxyestronsulfamat wird in dieser Kombination als bevorzugtes Sulfamat explizit beansprucht; es fallen aber zahlreiche weitere Steroid-3-sulfamate unter den Umfang der allgemeinen Formel. Als Wirkmechanismus für die erfindungsgemäßen pharmazeutischen Zusammensetzungen bzw. für die darin enthaltenen Steroid-3-sulfamate (bevorzugt mit mindestens einem 2-Alkoxysubstituenten) werden 1) Hemmung der Glucoseaufnahme in Tumorzellen, 2) Hemmung der Tumorangiogenese, 3) Abbau der Mikrotubuli; 4) Induzierung von Apoptose beschrieben. WO 00/76487 betrifft Stoffe, die die TNFα-induzierte Aromatase-Aktivität hemmen. Als solche werden 2-Alkoxyestron-3-sulfamate, bevorzugt 2-Methoxyestronsulfamat beansprucht.

WO 01/18028 beschreibt nicht-estrogene Estronsulfatase-inhibierende *N*-Acyl-18a-substituierte Steroid-3-sulfamate, wie z.B. 16α-Fluor-2-methoxy-18a-homoestradiol-(*N*-acetylsulfamat) bzw. 16α-Fluor-2-methoxy-18a-homoestron-(*N*-acetylsulfamat).

In Cancer 2000, 85, 983-994 werden die 2-Methoxyestradiol-, Docetaxel- und Paclitaxel-induzierte Apoptose in Hepatomazellen und deren Korrelation mit reaktiven Sauerstoffspezien verglichen.

Potter et. al. Int. J. Cancer 2000, 85, 584-589 untersuchen die Wirkung von 2-Methoxyestronsulfamat im Vergleich zu 2-Methoxyestron auf das Wachstum von Brustkrebszellen und induzierte Mammatumoren und finden, dass 2-Methoxyestronsulfamat ein beachtliches therapeutisches Potential zur Behandlung von Brustkrebs aufweist.

D'Amato et al., Proc. Natl. Acad. Sci., 91, 3964-3968, 1994, untersuchen die Inhibition der Angiogenese am Hühnerei durch den Test an der Chorion-Allantois-Membran und zeigen, dass durch 2-Methoxyestradiol die Tubolin-Polymerisation durch eine Wechselwirkung an der Colchicinbindungsstelle inhibiert wird.

Potter et. al. Molecular and Cellular Endocrinology 2000, 160, 61.66 untersuchen die Hemmung der Deoxyglucoseaufnahme in MCF-7-Brustkrebszellen durch 2-Methoxyestron und 2-Methoxyestron-3-sulfamat, die die Glucoseaufnahme um 25 bis 49% bei 10µM (ebenso 2-Methoxyestradiol und 2-Methoxyestron) hemmen, und folgern, dass die Verbindungen durch ihr Vermögen die Glucoseaufnahme zu hemmen, therapeutisches Potential zur Hemmung von Brustkrebs haben könnten.

Potter et. al. Cancer Research 2000, 60, 5441-5450 beschreibt 2-Methoxyestronsulfamat und 2-Ethoxyestronsulfamat als neue antimikrotubullenaktive Verbindungen, die *in vitro* Antikrebsaktivität in Mammakarzinomzellen aufwiesen, und daher auch eventuell *in vivo* aktiv sein könnten. In J. Steroid Biochem. and Mol. Biol. 1999, 69, 227-238 wird berichtet, dass die Hemmung der Steroidsulfatase-Aktivität ein wichtiger Ansatzpunkt bei der Behandlung von hormonabhängigen Mammakarzinomen ist. Explizit werden 2-Methoxyestronsulfamat, 17-Deoxyestronsulfamat und Estronsulfamat aufgeführt. Mono- bzw. bicyclische, nicht steroidale Sulfamate hemmen zwar die Steroidsulfatase, jedoch nicht so effektiv wie die entsprechenden Steroidderivate.

Die Aufgabe der vorliegenden Erfindung besteht darin, weitere Verbindungen zur Verfügung zu.stellen, die die Tubulinpolymerisation wirksam hemmen.

Die Aufgabe der vorliegenden Erfindung wird daher erfindungsgemäß durch die Verwendung von 2-substituierten Estra-1,3,5(10)-trien-3-yl sulfamaten der allgemeinen Formel I zur Herstellung eines Medikaments für die Behandlung von Tumorerkrankungen auf die die Inhibierung der Tubolinpolymerisation einen positiven Einfluß hat, gelöst: 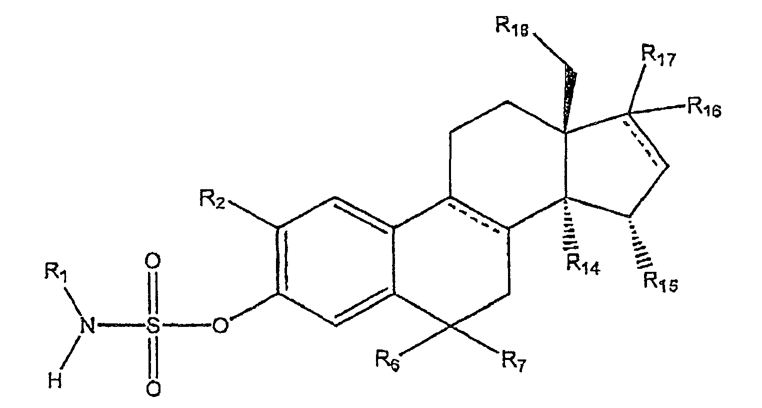 worin die Reste R¹ und R², R⁶ und R⁷, R¹⁴ und R¹⁵, R¹⁶ und R¹⁷ sowie R¹⁸ folgende Bedeutungen besitzen:
- R¹: Wasserstoff, C₁-C₅-Alkyl oder C₁-C₅-Acyl
- R²: C₁-C₅-Alkoxy, C₁-C₅-Alkyl oder einen Rest -O-CₙFₘHₒ, wobei n=1,2,3,4,5 oder 6, m>1 und m+o=2n+1 ist,
wenn R² ein Alkyl ist, kann R¹⁷ C₁-C₅-Alkoxy sein,
- R⁶: Wasserstoff
- R⁷: Wasserstoff, Hydroxy, Amino, Acylamino, wobei
wenn R⁶ und R⁷ kein Wasserstoff ist, dann kann R¹⁷ C₁-C₅-Alkoxy sein, oder
- R⁶ und R⁷: zusammen Sauerstoff, Oxim oder O(C₁-C₅-Alkyl)-Oxim darstellen
- R¹⁴ und R¹⁵: jeweils Wasserstoff oder zusammen eine Methylengruppe oder eine
zusätzliche Bindung,
- R¹⁶: Wasserstoff,
- R¹⁷: eine Gruppe CHXY ist, in der X für ein
Wasserstoffatom, ein Fluor, eine Hydroxygruppe oder einen Alkylrest mit 1 bis 4 Kohlenstoffatomen und Y für ein Wasserstoffatom oder ein Fluor steht, wobei,
wenn X ein Hydroxy ist, Y nur Wasserstoff sein kann
- R¹⁸: ein Wasserstoffatom, eine Methylgruppe
wenn R¹⁸ eine Methylgruppe ist, kann R¹⁷ Sulfamat SO₃NHR¹ sein,
wobei im B- und D-Ring des Steroidgerüstes die gestrichelten Linien zusätzlich bis zu zwei Doppelbindungen sein können,
sowie ihrer pharmazeutisch annehmbaren Salze.

Weiterhin umfasst die vorliegende Erfindung die neuen Substanzen als pharmazeutische Wirkstoffe, deren Herstellung, ihre therapeutische Anwendung und die pharmazeutischen Darreichungsformen, die die neuen Substanzen enthalten. Die erfindungsgemäßen Verbindungen der allgemeinen Formel (I) oder deren pharmazeutisch annehmbare Salze Werden für die Herstellung eines Arzneimittels zur Behandlung von Tumorerkrankungen, die sich durch die Hemmung der Tubulinpolymerisation positiv beeinflussen lassen, vorgeschlagen.

Es wurde festgestellt, dass die erfindungsgemäßen 2-substituierten Estra-1,3,5(10)-trien-3-yl sulfamaten *in vitro* die Tubulinpolymerisation überraschend stärker hemmen als 2-Methoxyestradiol selbst. Die erfindungsgemäßen Verbindungen hemmen die Proliferation von Tumorzellen und zeigen auch *in vivo* Antitumorwirkung. Zudem weisen die erfindungsgemäßen Verbindungen eine gute orale Bioverträglichkeit auf.

Unter Alkylresten sind gerad- oder verzweigtkettige, gesättigte oder ungesättigte Alkylreste zu verstehen. Als Vertreter für gerad- oder verzweigtkettige Alkylgruppen mit 1-5 Kohlenstoffatomen sind beispielsweise Methyl, Ethyl, Propyl, Isopropyl, Butyl, Isobutyl, tert.-Butyl, Pentyl, 1-Ethylpropyl, 1-Methylbutyl, 1,1-Dimethylpropyl; 2-Methylbutyl, 1,2-Dimethylpropyl, 3-Methylbutyl, 2,2-Dimethylpropyl zu nennen. Für ungesättigte Alkylreste stehen beispielsweise Allyl, Vinyl, Propenyl, Butenyl, aber auch Ethinyl, Propinyl oder Butinyl.

Acylreste bedeuten beispielsweise Formyl, Acetyl, Propionyl, Butyryl, iso-Butyryl oder Valeryl.

Für einen C₁-C₅-Alkoxyrest kann eine Methoxy-, Ethoxy-, n-Propoxy-, iso-Propoxy-, n-, iso-, tert.-Butoxy-, oder Pentoxygruppe stehen.

Bevorzugt gemäß vorliegender Erfindung ist die Verwendung solcher Verbindungen der allgemeinen Formel I, in denen:
- R¹: Wasserstoff oder C₁-C₅-Acyl,
- R²: Methoxy, Ethoxy oder 2,2,2-Trifluorethoxy,
- R⁶ und R⁷: jeweils Wasserstoff oder zusammen Oxim,
- R¹⁴ und R¹⁵: jeweils H oder zusammen eine Methylengruppe,
- R¹⁶: Wasserstoff,
- R¹⁷: Methyl, Difluormethyl, Hydroxymethyl,
- R¹⁸: Wasserstoff, Methyl
darstellen,
wobei im B- und D-Ring des Steroidgerüstes die gestrichelten Linien zusätzlich eine 8,9-oder eine 16,17-Doppelbindung bedeuten können.

Die Verwendung nachstehend genannter Verbindungen ist erfindungsgemäß besonders bevorzugt
1) 2-Ethyl-17β-methoxy-estra-1,3,5(10)-trien-3-yl sulfamat (3)
2) 2-Methoxy-17β-methyl-estra-1,3,5(10)-trien-3-yl sulfamat (5)
3) 17β-Difluormethyl-2-methoxy-estra-1,3,5(10)-trien-3-yl sulfamat (9)
4) 17β-Hydroxymethyl-2-methoxy-estra-1,3,5(10)-trien-3-yl sulfamat (11)
5) 2,17β-Dimethoxy-6-oximino-18a-homoestra-1,3,5(10)-trien-3-yl sulfamat
6) 17β-Ethoxy-2-methoxy-6-oximino-18a-homoestra-1,3,5(10)-trien-3-yl sulfamat

Gegenstand der vorliegenden Erfindung sind zudem 2-substituierte Estra-1,3,5(10)-trien-3-yl sulfamate der allgemeinen Formel I, zur Behandlung von Tumorerkrankungen, auf die die Inhibierung der Tubulinpolymerisation einen positiven Einfluß hat 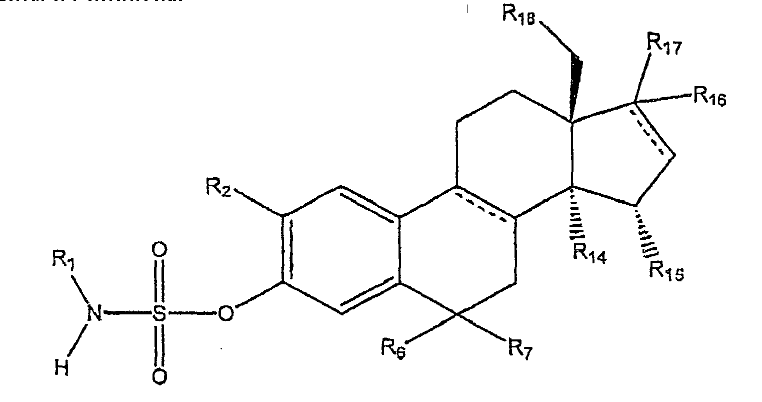 worin die Reste R¹ und R², R⁶ und R⁷, R¹⁴ und R¹⁵, R¹⁶ und R¹⁷ sowie R¹⁸ folgende Bedeutungen besitzen:
- R¹: Wasserstoff, C₁-C₅-Alkyl oder C₁-C₅-Acyl
- R²: C₁-C₅-Alkoxy,C₁-C₅-Alkyl oder einen Rest -O-CₙFₘHₒ,
wobei n=1,2,3,4,5 oder 6, m>1 und m+o=2n+1 ist,
wenn R² ein Alkyl ist, kann R¹⁷ C₁-C₅-Alkoxy sein,
- R⁶: Wasserstoff
- R⁷: Wasserstoff, Hydroxy, Amino, Acylamino
wenn R⁶ und R⁷ kein Wasserstoff ist, dann kann R¹⁷ C₁-C₅-Alkoxy sein oder
- R⁶ und R⁷: zusammen Sauerstoff, Oxim oder O(C₁-C₅-Alkyl)-Oxim darstellen
- R¹⁴ und R¹⁵: jeweils Wasserstoff oder zusammen eine Methylengruppe oder eine zusätzliche
Bindung,
- R¹⁶: Wasserstoff,
- R¹⁷: eine Gruppe CHXY ist, in der X für ein
Wasserstoffatom, ein Fluor, eine Hydroxygruppe oder einen Alkylrest mit 1 bis 4 Kohlenstoffatomen und Y für ein Wasserstoffatom oder ein Fluor steht, wobei,
wenn X ein Hydroxy ist, Y nur Wasserstoff sein kann
- R¹⁸: ein Wasserstoffatom, eine Methylgruppe, wobei wenn R¹⁸ eine Methylgruppe ist, kann
- R¹⁷: Sulfamat SO₃NHR¹ sein,
wobei im B- und D-Ring des Steroidgerüstes die gestrichelten Linien zusätzlich bis zu zwei Doppelbindungen sein können, sowie ihre pharmazeutisch annehmbaren Salze.

Die verschiedenen Alkyl-, ungesättigten Alkyl-, Acyl-, Alkoxyl-Reste entsprechen denen, die für die erfindungsgemäße Verwendung aufgezählt sind.

Auch die bei den Verbindungen bevorzugten Substituenten, entsprechen denen, die bei der erfindungsgemäßen Verwendung genannt sind.

Die nachstehend genannten Verbindungen sind erfindungsgemäß besonders bevorzugt
1) 2-Ethyl-17β-methoxy-estra-1,3,5(10)-trien-3-yl sulfamat
2) 2-Methoxy-17β-methyl-estra-1,3,5(10)-trien-3-yl sulfamat
3) 17β-Difluormethyl-2-methoxy-estra-1,3,5 (10)-trien-3-yl sulfamat
4) 17β-Hydroxymethyl-2-methoxy-estra-1,3,5(10)-trien-3-yl sulfamat
5) 2,17β-Dimethoxy-6-oximino-18a-homoestra-1,3,5(10)-trien-3-yl sulfamat
6) 17β-Ethoxy-2-methoxy-6-oximino-18a-homoestra-1,3,5(10)-trien-3-yl sulfamat

### Pharmakologische Daten

### 1. Hemmung der Tubulinpolymerisation

Die erfindungsgemäßen Verbindungen wurden in verschiedenen Modellen getestet. Die erfindungsgemäßen Verbindungen der allgemeinen Formel I zeichnen sich dadurch aus, dass sie die Tubulinpolymerisation stärker hemmen als 2-Methoxyestradiol. Die *in vitro* Testung der Tubulinpolymerisationsbeeinflussung wurde folgendermaßen durchgeführt:

Mikrotubuläres Protein wurde nach Shelanski et. al. (Shelanski et. al. Proc. Natl. Acad. Sci. USA 1973, 70, 765-8) über zyklische Assemblierung / Deassemblierung aus Schweinehirn gereinigt. Das verwendete Buffersystem hatte folgende Zusammensetzung: 20 mM PIPES (1,4-Piperazine-diethane-sulfonsäure, pKa 6,8), 80 mM NaCl, 0,5 mM MgCl₂, 1 mM EGTA (Ethylenglykol-bis-(2-aminoethyle)-tetraessigsäure). Für die Wirkstofftestung wurden Proteinkonzentrationen von 1 mg/ml (ca. 10⁻⁵ mM Tubulin) eingesetzt. Die Proteinbestimmung erfolgte nach der Lowry-Methode (Lowry et al. J. Biol. Chem. 1951, 193, 265-75) mit Rinderserumalbumin als Standard. Die Assemblierung von Mikrotubuli erfolgte in Gegenwart von 0.25 mM GTP und Erwärmen der Proben auf 37 °C.

Die Mikrotubulusbildung wurde mit Hilfe der Turbidimetrie bei einer Wellenlänge von 340 nm geprüft. Der Gleichgewichtszustand, bei dem das mikrotubuläre Protein keinen Zuwachs der Assemblatkonzentration (entsprechend der Mikrotubuluskonzentration) und der Trübungswert keinen Anstieg mehr aufweist, wird typischerweise nach 20 Minuten erreicht.

Die Testung der Wirkstoffe erfolgte durch deren Zugabe am Anfang der Assemblierung oder im Gleichgewichtszustand. Abweichungen der Trübungskurven von der Kontrolle charakterisieren ihre Wirksamkeit. Zur Wirkungskontrolle und Bewertung der Trübungsmesswerte wurde stets eine Transmissionselektronenmikroskopische Untersuchung (CEM 902 A, Zeiss /Oberkochen) der Assemblate nach Negativfärbung mit 1 %igem wässrigen Uranylazetat ausgeführt.

**Tab. 1.**

| Name | Hemmung der Tubulinpolymerisation IC₅₀[µM] |
|---|---|
| 2-Methoxyestradiol | 2,70 |
| 2-Methoxy-estra-1,3,5(10)-trien-3-yl sulfamat * **(1)** | 0,67 |
| 2-Methoxy-17(20)-methylen-estra-1,3,5(10)-trien-3-yl sulfamat * **(4)** | 1,4 |
| 2-Ethyl-17β-methoxy-estra-1,3,5(10)-trien-3-yl sulfamat **(3)** | 1,4 |
| 17β-Difluormethyl-2-methoxy-estra-1,3,5 (10)-trien-3-yl sulfamat **(9)** | 1,5 |
| 17(20)-Difluormethylen-2-methoxy-estra-1,3,5(10)-trien-3-yl sulfamat * **(8)** | 1,10 |
| 2-Methoxy-17β-methyl-estra-1,3,5(10)-trien-3-yl sulfamat **(5)** | 1,30 |

| | |
|---|---|
| * Vergleichsverbindung | |

### 2. Hemmung der Zellproliferation

Die erfindungdgemäßen Verbindungen zeichnen sich durch eine potente Hemmung der Zellproliferation aus.

Zellkulturen der folgenden Zellinien wurden in 96well Mikrotiterplatten angelegt:
1. MaTu/ADR Multidrug-resistente humanen Brusttumorzellen (Epo GmbH Berlin), 5000 Zellen/Weil.
2. HCT116 humane Kolontumorzellen (ATCC CCL-247); 3000 Zellen/Well.
3. NCl-H460 humane nicht-kleinzelliges Lungenkarzinomzellen (ATCC HTB-177), 3000 Zellen/Well.
4. DU145 humane Prostatatumorzellen (ATCC HTB-81), 5000 Zellen/Well.
5. HMVEC humane primäre dermale mikrovaskuläre Endothelzellen, 7500 Zellen/Well.

Nach 24 stündiger Inkubation in einem Zellkulturbrutschrank bei 37°C wurden die Zellen einer Mikrotiterplatte mit Kristallviolett gefärbt (Referenzplatte), während die Zellen in den Testplatten für 4 Tage mit den Testsubstanzen in den Konzentrationen 0.1 - 10 µM, sowie mit dem Lösungsmittel DMSO alleine (Lösungsmittelkontrolle) , inkubiert wurden. Die Zellproliferation wurde durch Färbung der Zellen mit Kristallviolett bestimmt. Die Extinktion des Kristallvioletts wurde photometrisch bei 595 nm bestimmt. Die Prozentzahl der Änderung der Zellzahl in den Testplatten wurde nach Normalisation der Extinktionswerte auf die Referenzplatte (0%) und auf die Lösungsmittelkontrollen (100%) bestimmt. Die halbmaximale Inhibition des Zellwachstums (IC50) wurde als die Substanzkonzentration bestimmt, bei der 50% der Zellzahl der Lösungsmittelkontrollen vorhanden waren.

**Tab. 2**

| Name | Hemmung der Zellproliferation IC50 [µM] | | | | |
|---|---|---|---|---|---|
| | NCI-H460 | HCT116 | DU145 | MaTu/ADR | HMVEC |
| Taxol | 0,004 | 0,004 | 0,004 | 0,4 | 0,004 |
| 2-Methoxyestradiol | 1,8 | 1,1 | 1,9 | 0,2 | 2 |
| 2-Methoxy-17(20)-methylen-estra-1,3,5(10)-trien-3-yl sulfamat * **(4)** | 0,18 | 0,18 | 0,18 | <0,1 | 0,16 |
| 2-Methoxy-estra-1,3,5(10)-trien-3-yl sulfamat * **(1)** | 0,4 | 0,4 | 0,5 | 0,11 | <0,1 |
| 2-Ethyl-17β-methoxy-estra-1,3,5(10)-trien-3-yl sulfamat **(3)** | 0,18 | 0,18 | 0,18 | 0,12 | 0,16 |
| 17β-Difluormethyl-2-methoxy-estra-1,3,5(10)-trien-3-yl sulfamat **(9)** | 0,22 | 0,3 | 0,4 | 0,11 | 0,2 |
| 17(20)-Difluormethylen-2-methoxy-estra-1,3,5(10)-trien-3-yl sulfamat * **(8)** | 0,22 | 0,3 | 0,5 | 0,17 | 0,2 |
| 17β-Hydroxymethyl-2-methoxy-estra-1,3,5(10)-trien-3-yl sulfamat **(11)** | 0,18 | 0,18 | 0,2 | <0,1 | <0,1 |
| 2-Methoxy-17(20)-methylen-6-oximino-estra-1,3,5(10)-trien-3-yl sulfamat * **(7)** | 0,5 | 0,5 | 0,55 | 0,13 | 0,13 |

| | | | | | |
|---|---|---|---|---|---|
| * Vergleichsverbindung | | | | | |

### Dosierung

Im allgemeinen sind zufriedenstellende Resultate zu erwarten, wenn die täglichen Dosen einen Bereich von 5 µg bis 50 mg der erfindungsgemäßen Verbindung pro kg Körpergewicht umfassen. Bei größeren Säugetieren, beispielsweise dem Menschen, liegt eine empfohlene tägliche Dosis im Bereich von 10 µg bis 30 mg pro kg Körpergewicht.

Geeignete Dosierungen für die erfindungsgemäßen Verbindungen betragen von 0,005 bis 50 mg pro Tag pro kg Körpergewicht, je nach Alter und Konstitution des Patienten, wobei die notwendige Tagesdosis durch Einmal- oder Mehrfachabgabe appliziert werden kann.
Aufgrund der besonderen Depotwirkung der Estrogen-Sulfamate können die erfindungsgemäßen Verbindungen aber auch in größeren Abständen als einmal am Tag verabreicht werden

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmittein, Verdünnungsmitteln, Geschmackskorrigentien, Färbemitteln usw. verarbeitet und in die gewünschte Applikationsform überführt. Dabei ist auf Remington's Pharmaceutical Science, 15th ed. Mack Publishing Company, East Pennsylvania (1980) hinzuweisen.

Für die orale Applikation kommen insbesondere Tabletten, Dragees, Kapseln, Pillen, Pulver, Granulate, Pastillen, Suspensionen, Emulsionen oder Lösungen in Frage.
Für die parenterale Applikation sind Injektion- und Infusionszubereitungen möglich. Für die intraartikulären Injektion können entsprechend zubereitete Kristallsuspensionen verwendet werden.

Für die intramuskuläre Injektion können wässrige und ölige Injektionslösungen oder Suspensionen und entsprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen als auch zur lokalen Therapie verwendet werden.

Zur pulmonalen Applikation der neuen Verbindungen können diese in Form von Aerosolen und Inhalaten verwendet werden.

Für die topische Auftragung sind Formulierungen in Gele, Salben, Fettsalben, Cremes, Pasten, Puder, Milch und Tinkturen möglich. Die Dosierung der Verbindungen der allgemeinen Formel I sollte in diesen Zubereitungen 0,01% - 20% betragen um eine ausreichende pharmakologische Wirkung zu erzielen.

Die vorliegende Erfindung umfasst die Verwendung der erfindungsgemäßen Verbindungen der allgemeinen Formel I zur Herstellung eines Arzneimittels, insbesondere zur Behandlung von Tumorerkrankungen, die sich durch die Hemmung der Tubulinpolymerisation positiv beeinflussen lassen.

Die erfindungsgemäßen Verbindungen der allgemeinen Formel I werden bevorzugt verwendet zur Herstellung eines Arzneimittels zur Behandlung von Tumorerkrankungen der männlichen und weiblichen Keimdrüsen, männlichen und weiblichen Sexualorgane einschließlich der Brustdrüsen, insbesondere von Prostatakarzinomen oder Mammakarzinom.

Ferner betrifft die vorliegende Erfindung pharmazeutische Zusammensetzungen, die mindestens eine erfindungsgemäß besonders bevorzugte Verbindung, gegebenenfalls in Form eines pharmazeutisch/pharmakologisch verträglichen Salzes, ohne oder zusammen mit pharmazeutisch verträglichen Hilfs- und/öder Trägerstoffen enthalten.

Diese pharmazeutischen Zusammensetzungen und Arzneimittel können zur oralen, rektalen, vaginalen, subkutanen, perkutanen, intravenösen oder intramuskulären Applikation vorgesehen sein. Sie enthalten neben üblichen Träger- und/oder Verdünnungsmitteln mindestens eine erfindungsgemäß besonders bevorzugte Verbindung.
Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder auch Depotformen.

Die pharmazeutischen Zusammensetzungen enthaltend mindestens eine der erfindungsgemäßen Verbindungen werden bevorzugt oral appliziert.

Es kommen auch parenterale Zubereitungen wie Injektionslösungen in Betracht. Weiterhin seien als Zubereitungen beispielsweise auch Suppositorien und Mittel zur vaginalen Anwendung genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Verbindungen der allgemeinen Formel I können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten.

Die Verbindungen der allgemeinen Formel I enthaltende Kapseln können beispielsweise hergestellt werden, indem man die Verbindung(en) der allgemeinen Formel I mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyethylenglykol bzw. deren Derivaten herstellen.

Die erfindungsgemäßen Verbindungen können zur Therapie von Prostatakarzinomen mit einem oder mehreren der folgenden Wirkstoffen kombiniert verabreicht werden:
1) Antiandrogene wie CPA, Flutamid, Casodex etc.
2) Gonadrotrophormon (GnRH) Agonisten
3) 5α-Reduktase Hemmer wie Finasterid
4) Zytostatika
5) VEGF-Kinase-Inhibitoren
6) Antigestagene
7) Antiestrogene
8) Antisense Oligonukleotide
9) EGF-Antikörper
10) Estrogene

Darüber hinaus können die erfindungsgemäßen Verbindungen der allgemeinen Formel I zur Therapie und Prophylaxe weiterer oben nicht genannter Krankheitszustände eingesetzt werden.

Die erfindungsgemäßen Verbindungen der allgemeinen Formel I lassen sich wie nachstehend beschrieben herstellen:

### Allgemeiner Syntheseteil

Die Funktionalisierung des C-Atoms 2 eines Estra-1,3,5(10)-trien-17-on-derivates erfolgt vorzugsweise durch Friedel-Crafts-Acylierung wie in der Literatur beschrieben (T. Nambara et al. Chem. Pharm. Bull. 1979, 18, 474-480).

Nach Wechsel der Schutzgruppe in Position 3 wird durch Baeyer-Villiger-Oxidation ein 2-Carboxy-estra-1,3,5(10)-trien-17-on generiert (M.B. Smith, J. March, March's Advanced Organic Chemistry, 5. Edition, Wiley Sons 2001, 1417-1418 und dort zit. Lit.). Der Ester wird verseift und mit dem entsprechenden Alkylhalogenid unter basischen Bedingungen in einen 2-Alkylether überführt. Alternativ kann nun das 17-Keton wie bekannt reduziert und verethert werden. Die Spaltung der Schutzgruppe in Position 3 erfolgt wie in der Literatur beschrieben (T.W. Greene, P.G.M. Wuts, Protective Groups in Organic Synthesis, Wiley & Sons, 1999, 249-275). Dieses Verfahren oder andere aus der Literatur bekannte (P.N. Rao, J.W. Cessac, Steroids 2002, 67, 1065-1070 und dort zit. Lit.) sind entsprechend auf die 18a-Homoderivate anwendbar.

Die vorzugsweise durch Friedel-Crafts-Acylierung erhaltenen 2-Acylderivate können durch Reduktion mit Natriumborhydrid und anschließender Hydrierung in die entsprechenden 2-Alkylderivate übergeführt werden.

Die 2-Hydroxylierung, ausgehend von Verbindungen der allgemeinen Formel II (R₂ = H), 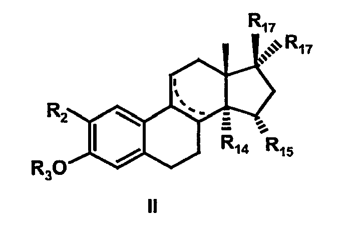 bei denen R₁₄ und R₁₅ zusammen eine Methylenbrücke bilden bzw. welche zusätzliche Doppelbindungen im Steroidgerüst besitzen, wird durch ortho-Metalierung realisiert, wobei für R₃ als ortho-dirigierende Schutzgruppe vorzugsweise eine Ether-(z.B. H.E. Paaren, S.R. Duff, US6448419 und dort zit. Lit.) oder Carbamatschutzgruppe (V. Snieckus, Chem. Rev. 1990, 90, 879-933) verwendet wird. Die elektrophile Substitution erfolgt nach 2-Lithiierung mit Trialkylborat und anschließender basischer Oxidation mit Wasserstoffperoxid. Die selektiv erhaltene 2-Hydroxygruppe kann anschließend in bekannter Weise (Z. Wang, M. Cushman, Synth. Commun. 1998, 28, 4431) zur 2-Alkoxyverbindung umgewandelt und entschützt werden. Anschließende Oppenauer-Oxidation (C. Djerassi, Org. React. 1951, 6, 207, S. Schwarz et al. Pharmazie 2001, 56, 843-849) liefert die 17-Ketoverbindungen, welche weiter funktionalisiert und wie bekannt zu den Sulfamaten umgesetzt werden können.

Ausgehend von den 2-funktionalisierten 17-Keto-Derivaten können 17-Oxirane (M. Hübner, I. Noack, J. prakt. Chem. 1972, 314, 667), und daraus die entsprechenden 17-Formylderivate (M. Hübner, K. Ponsold, Z. Chem. 1982, 22, 186) bzw. 17-monofluorierten Methylderivaten (B. Menzenbach et al. DE10043846) hergestellt werden.

Ebenso können aus den 2-funktionalisierten Derivaten die entsprechenden 17-Oxim-, 17-Alkylen- (sog. Wittig-Reaktion, siehe z.B S. Schwarz et al. Pharmazie 2001, 56, 843-849), 17-Difluormethylen- (Wadsworth-Emmons-Reaktion, S.R. Piettre, L. Cabanas, Tetrahedron Lett. 1996, 37, 5881-4884), 17-Deoxo-, 17β-Alkyl-, 17β-Hydroxymethylderivate hergestellt (z.B. R.H. Peters et al., J. Med. Chem. 1989, 32, 1642; G.E. Agoston et al. WO02/42319 und anschließend in 3-Position sulfamoyliert werden.

Nach Cushman et al. (J. Med. Chem. 1997, 40, 2323) erfolgt die Synthese 6-funktionalisierter Estrogenderivate durch Oxidation des Acetyl-geschützen Estrogenderivates mit Chromtrioxid.

17-Fluorierte Derivate können aus den entsprechenden 17-Oxo bzw. 17-Hydroxy-Derivaten mit Diethylamino-schwefeltrifluorid hergestellt (M. Hudlicky, Org. Reactions 1988, 35, 513; J.T. Welch, Fluorine in Bioorganic Chemistry 1991, John Wiley, New York; S. Rozen et al. Tetrahedron Lett. 1979, 20, 1823-1826) und anschließend sulfamoyliert werden.

Die vorliegende Erfindung wird anhand der nachfolgenden Beispiele näher erläutert, ohne sie darauf einzuschränken:

### Herstellungsverfahren

### Allgemeine Synthesevorschrift 1 zur Herstellung von Sulfamaten

Es werden ein Äquivalent eines Estra-1,3,5(10)-trien-Derivates in Ethylenchlorid unter Rühren gelöst bzw. suspendiert und mit 5 Äquivalenten 2,6-Di-tert.-butylpyridin versetzt. Anschließend werden unter Argon 10 Äquivalente Sulfamoylchlorid zugegeben und bei Raumtemperatur gerührt. Die Lösung wird bis zum vollständigen Umsatz gerührt (DC-Kontrolle, 1-5h) und dann mit Wasser versetzt. Bei säureempfindlichen Verbindungen wird vorher mit rund 10 Äquivalenten Triethylamin gepuffert. Die wässrige Phase wird mehrmals mit Dichlormethan oder Essigester extrahiert. Die vereinigten organischen Phasen werden über Natriumsulfat getrocknet und am Vakuum eingeengt und anschließend flashchromatographisch gereinigt.

### Allgemeine Synthesevorschrift 2 zur Acylierung von Sulfamaten

Ein Äquivalent des Estra-1,3,5(10)-trien-Sulfamates bzw. Bissulfamates werden in Pyridin gelöst und unter Eiskühlung (0 bis 5°C) mit 5 Äquivalenten Anhydrid versetzt. Es wird 1h bei Raumtemperatur weitergerührt und dann mit Wasser versetzt. Die wässrige Phase wird mehrmals mit Dichlormethan oder Essigester extrahiert. Die vereinigten organischen Phasen werden mit 6N Salzsäure und anschließend mit Wasser und Natriumchloridlösung gewaschen. Danach wird über Natriumsulfat getrocknet und am Vakuum eingeengt und anschließend flashchromatographisch gereinigt.

### Beispiel 1 (Vergleichsbeispiel)

### 2-Methoxy-estra-1,3,5(10)-trien-3-yl sulfamat (1):

1.48 g 3-Hydroxy-2-methoxy-estra-1,3,5(10)-trien wurden nach der allgemeinen Synthesevorschrift 1 zum Produkt umgesetzt und anschließend durch Flashchromatographie (Toluol/Essigester = 19:1 → 10:1) gereinigt. Es wurden 1.69 g (89%) 2-Methoxy-estra-1,3,5(10)-trien-3-yl sulfamat (**1**) als amorphes Pulver erhalten. ¹H-NMR (CDCl₃): δ = 0.75 (s, 3H; 18-CH3₎, 2.76-2.84 (m, 2H; 6-CH₂), 3.87 (s, 3H; 2-OCH₃), 4.96 (s, 2H; NH₂), 6.94, 7.03 (2 s, 2H; 1-H, 4-H).

### Beispiel 2 (Vergleichsbeispiel)

### 17α-Allyy-17β-hydroxy-2-methoxy-estra-1,3,5(10)-trien-3-yl sulfamat (2a) und 2-Methoxy-17-(E-vinyl)methylen-estra-1,3,5(10)-trien-3-yl sulfamat (2b):

622 mg 3-Hydroxy-2-methoxy-estra-1,3,5(10)-frien-17-on wurden in 35 mL abs. Tetrahydrofuran unter Argon gelöst und bei -70°C mit 20 mL Allylmagnesiumbromldlösung (1M in Diethylether) versetzt. Anschließend ließ man auf Raumtemperatur kommen und goss nach 3h auf wässr. Ammoniumchloridlsg. und extrahierte mit Essigester (2x). Die vereinigten organischen Phasen wurden mit ges. Kochsalzisg. gewaschen, getrocknet und am Rotationsverdampfer eingeengt. Flashchromatographie (Cyclohexan/Essigester = 5:1 → 3:1) lieferte 663 mg (94%) 17α-Allyl-3,17β-dihydroxy-2-methoxy-estra-1,3,5(10)-trien als farblose Kristalle.
¹H-NMR (CDCl₃): δ = 0.94 (s, 3H; 18-CH₃), 3.86 (s, 3H; 2-OCH₃), 5.16-5.23 (m, 1H; C=CH₂), 5.44 (s, 1H; OH), 5.96-6.06 (m, 1H; -CH=C), 6.63, 6.79 (2 s, 2H; 1-H, 4-H).

104 mg 17α-Allyl-3,17β-dihydroxy-2-methoxy-estra-1,3,5(10)-trien wurden nach der allgemeinen Synthesevorschrift 1 zu den Produkten umgesetzt und anschließend durch Flashchromatographie (Cyclohexan/Essigester = 15:1 → 10:1 → 5:1) gereinigt. Neben Ausgangsmaterial und wassereliminiertem Ausgangsmaterial wurden 28 mg (22%) 17α-Allyl-17β-hydroxy-2-methoxy-estra-1,3,5(10)-trien-3-yl sulfamat (**2a**) sowie 36 mg (29%) 2-Methoxy-17-(E-vinyl)methylen-estra-1,3,5(10)-trien-3-yl sulfamat (**2b**) als amorphe Pulver erhalten.
**2a:** ¹H-NMR (CDCl₃): δ = 0.94 (s, 3H; 18-CH₃), 2.78-2.82 (m, 2H; 6-CH₂), 3.87 (s, 3H; 2-OCH₃), 5.01 (s, 2H; NH₂), 5.16-5.24 (m, 1H; C=CH₂), 5.95-6.07 (m, 1H; -CH=C), 6.92, 7.03 (2 s, 2H; 1-H, 4-H).
**2b:** ¹H-MMR (CDCl₃): δ = 0.83 (s, 3H; 18-CH₃), 2.77-2.84 (m, 2H; 6-CH₂), 3.87 (s, 3H; 2-OCH₃), 4.98-5.13 (m, 4H, 22-CH₂, NH₂), 5.76 (d, *³J* = 10.8 Hz, 1H; 20-CH=), 6.40-6.50 (m, 1H; 21-CH=), 6.94, 7.03 (2 s, 2H; 1-H, 4-H)

### Beispiel 3:

### 2-Ethyl-17β-methoxy-estra-1,3,5(10)-trien-3-yl sulfamat (3):

362 mg 2-Acetyl-3-benzyloxy-estra-1,3,5(10)-trien-17-on wurden in 150 mL Tetrahydrofuran/Methanol 10/1 gelöst, mit Natriumborhydrid versetzt und 3 h bei Raumtemperatur gerührt, mit Essigsäure versetzt und am Rotationsverdampfer eingeengt. Der· Rückstand wurde mit Wasser versetzt und mit Dichlormethan extrahiert (2x). Die vereinigten organischen Phasen wurden mit ges. Natriumbicarbonatisg. gewaschen, getrocknet und am Rotationsverdampfer eingeengt. Der Rückstand wurde in 5 mL abs. Dimethylformamid unter Argon gelöst, mit 0.32 g Natriumhydrid (∼ 50%-ig) und anschließend mit 1.5 mL Methyliodid versetzt und über Nacht bei Raumtemperatur gerührt. Nach Zugabe von Wasser wurde mit Essigester extrahiert (2x). Die vereinigten organischen Phasen wurden mit Wasser und anschließend mit ges. Kochsalzisg. gewaschen, getrocknet und am Rotationsverdampfer eingeengt. Aashchromatographie (Toluol/Essigester = 60:1) lieferte 301 mg (77%) 2-(1-Methoxyethyl)-3-benzyloxy-17β-methoxy-estra-1,3,5(10)-trien als farblose Kristalle.

290 mg 2-(1-Methoxyethyl)-3-benzyloxy-17β-methoxy-estra-1,3,5(10)-trien wurden in je 20 mL Essigester und Dichlormethan gelöst und dann mit 3 Tropfen Essigsäure und 110 mg Palladium auf Aktivkohle (10%-ig) versetzt. Die Hydrierung erfolgte unter Normaldruck über 8h. Der Katalysator wurde abfiltriert und die Lösung am Rotationsverdampfer eingeengt und mehrmals mit Toluol coevaporiert. Flashchromatographie (Toluol/Aceton = 80:1) lieferte 220 mg (98%) 2-Ethyl-3-hydroxy-17β-methoxy-estra-1,3,5(10)-tfien als farblosen Schaum.
¹H-NMR (CDCl₃): δ = 0.79 (s, 3H; 18-CH₃), 1.22 (t, *³J* = 7.6 Hz, 3H; CH₃). 2.59 (q, *³J* = 7.6 Hz, 2H; 2-CH₂Me), 2.76-2.79 (m, 2H; 6-CH₂), 3.13 (t, *³J* ≈ 8.4 Hz, 1H; 17α-H), 3.38 (s, 3H; 17β-OCH₃), 4.57 (s, 1H; OH), 6.48, 7.04 (2 s, 2H; 1-H, 4-H).

93 mg 2-Ethyl-3-hydroxy-17β-methoxy-estra-1,3,5(10)-frien wurden nach der allgemeinen Synfhesevorschrifi 1 zum Produkt umgesetzt und anschließend durch Flashchromatographie (Toluol/Essigester = 20:1 → 10:1) gereinigt. Es wurden 110 mg (94%) 2-Ethyl-17β-methoxy-estra-1,3,5(10)-trien-3-yl sulfamat (3) als farblose Kristalle erhalten.
¹H-NMR (CDCl₃): δ = 0.78 (s, 3H; 18-CH₃). 1.22 (t, *³J* = 7.6 Hz, 3H; CH₃), 2.69 (q, *³J* = 7.6 Hz**,** 2H; 2-CH₂Me), 2.81-2.84 (m, 2H; 6-CH₂), 3.31 (t, *³J* ≈ 8.2 Hz, 1H; 17α-H), 3.37 (s, 3H; 17β-OCH₃), 4.97 (s, 2H; NH₂), 7.07, 7.18 (2 s, 2H; 1-H, 4-H).

### Beispiel 4 (Vergleichsbeispiel)

### 2-Methoxy-17(20)-methylen-estra-1,3,5(10)-trien-3-yl sulfamat (4):

Zu einer Lsg. aus 11.8 g Methyltriphenylphosphoniumbromid in 50 mL abs. Dimethylsulfoxid wurden bei Raumtemperatur 1,6 g Natriumhydrid (55%-ig) gegeben. Nach 30 min wurde dazu eine Lsg. aus 1.00 g 3-Hydroxy-2-methoxy-estra-1,3,5(10)-trien-17-on in 50 mL abs. Dimethylsulfoxid zugegeben und anschließend auf 70°C erwärmt Nach 1 h wurde auf Raumtemperatur abgekühlt, mit Wasser versetzt und mit Diethylether extrahiert (3x). Die vereinigten organischen Phasen wurden mit Wasser gewaschen, getrocknet und am Rotationsverdampfer eingeengt. Flashchromatographie (Toluol/Essigester = 40:1) lieferte 979 mg (98%) 3-Hydroxy-2-methoxy-17(20)-methylen-estra-1,3,5(10)-trien als farblose Kristalle.
¹H-NMR (CDCl₃): δ = 0.83 (s, 3H; 18-CH₃), 3.86 (s, 3H; 2-OCH₃), 4.67 (t, 1H; =CH₂), 5.41 (s, 1H; 3-OH), 6.64, 6.80 (2 s, 2H; 1-H, 4-H).

100 mg 3-Hydroxy-2-methoxy-17(20)-methylen-estra-1,3,5(10)-trien wurden nach der allgemeinen Synthesevorschrift 1 (Base jedoch im Überschuss bzgl. Sulfamoylchlorid) zum Produkt umgesetzt und anschließende durch Flashchromatographie (Toluol/Essigester = 15:1 → 10:1) gereinigt. Es wurden 90 mg (72%) 2-Methoxy-17(20)-methylen-estra-1,3,5(10)-trien-3-yl sulfamat (4) als farbloses Pulver erhalten.
¹H-NMR (CDCl₃): δ = 0.83 (s, 3H; 18-CH₃), 2.79-2.81 (m, 2H; 6-CH₂), 3.87 (s, 3H; 2-OCH₃), 4.68 (t, *²J* = 1.2 Hz, 2H; 20-CH₂), 5.01 (s, 2H; NH₂), 6.94, 7.03 (2 s, 2H; 1-H, 4-H).

### Beispiel 5

### 2-Methoxy-17β-methyl-estra-1,3,5(10)-trien-3-yl sulfamat (5):

108 mg 3-Hydroxy-2-methoxy-17β-mathyl-estra-1,3,5(10)-trien (erhalten durch Hydrierung von 3-Hydroxy-2-methoxy-17(20)-methyten-estra-1,3,5(10)-trien) wurden nach der allgemeinen Synthesevorschrift 1 zum Produkt umgesetzt und anschließend durch,Flashchromatographie (Toluol/Essigester = 30:1 → 15:1) gereinigt. Es wurden 124 mg (91%) 2-Methoxy-17β-methyl-estra-1,3,5(10)-trien-3-yl sulfamat **(5)** als farblose Kristalle erhalten.
¹H-NMR (CDCl₃): δ = 0.59 (s, 3H; 18-CH₃), 0.88 (d, *³J* = 7.0 Hz, 3H; 17-CH₃), 2.77-2.80 (m, 2H; 6-CH₂), 3.86 (s, 3H; 2-OCH₃), 5.01 (s, 2H; NH₂), 6.93, 7.02 (2 s, 2H; 1-H, 4-H).

### Beispiel 6 (Vergleichsbeispiel)

### 2-Methoxy-17(20)-methylen-6-oxo-estra-1,3,5(10)-trien-3-yl sulfamat (6):

3-Acetoxy-2-methoxy-17(20)-methyfen-6-oxo-estra-1,3,5(10)-trien wurde in 6-Position mit Chromtrioxid in Essigsäure bei 10°C in einer Ausbeute von 48% oxidiert und anschließend die Acetylgruppe durch Natriummethanolat in Methanol quantitativ abgespalten. Sulfamoyllerung von 72 mg dieses Zwischenproduktes nach der allgemeinen Synthesevorschrift 1 und anschließende Flashchromatographie (Toluol/Essigester = 5:1) lieferte 2-Methoxy-17(20)-methylen-6-oxo-estra-1,3,5(10)-trien-3-yl sulfamat (**6**) in 75% Ausbeute als farblose Kristalle.
¹H-NMR (CDCl₃): δ = 0.83 (s, 3H; 18-CH₃), 3.98 (s, 3H; 2-OCH₃), 4.71 (s, 2H; =CH₂), 5.23 (s, 2H; NH₂), 7.00, 7.98 (2 s, 2H; 1-H, 4-H).

### Beispiel 7 (Vergleichsbeispiel)

### 2-Methoxy-I7(20)-rnethylen-6-oxlrnirio-estra-1,3,5(I0)-trien-3-yl sulfamat (7):

55 mg 2-Methoxy-17(20)-methylen-6-oxo-estra-1,3,5(10)-trien-3-yl sulfamat (**6**) wurden mit 70 mg Hydroxylamin-hydrochlorid und 100 mg Natriumhydrogencarbonat in 3 mL Methanol für 3 h auf 60°C erhitzt. Anschließend wurde auf Raumtemperatur abgekühlt, mit Wasser versetzt und mit Essigester extrahiert (3x). Die vereinigten organischen Phasen wurden mit ges. Natriumchloridisg. gewaschen, getrocknet und am Rotationsverdampfer eingeengt. Flashchromatographie (Toluol/Aceton = 8:1 → 5:1) lieferte 26 mg (46%) 2-Methoxy-17(20)-methylen-6-oximino-estra-1,3,5(10)-trien-3-yl sulfamat (**7**) als weißen amorphen Feststoff sowie als Nebenprodukt 3-Hydroxy-2-methoxy-17(20)-methylen-6-oximino-estra-1,3,5(10)-trien.
¹H-NMR (Aceton-d₆): δ = 0.71 (s, 3H; 18-CH₃), 3.78 (s, 3H; 2-OCH₃), 4.55 (d, 2H; =CH₂), 6.94, 7.75 (2 s, 2H; 1-H, 4-H).

### Beispiel 8 (Vergleichsbeispiel)

### 17(20)-Difluormethylen-2-methoxy-estra-1,3,5(10)-trien-3-yl sulfamat (8):

Zu einer Lsg. aus 1.53 mL Diethyl(difluormethyl)phosphonat in 10 mL abs. 1,2-Dimethoxyethan wurden bei -70°C 6.4 mL einer tert.-Butyllithiumlösung (1.5M in Pentan) unter Rühren zugetropft. Nach 20 min wurde dazu eine Lsg. aus 733 mg 3-Hydroxy-2-methoxy-estra-1,3,5(10)-trien-17-on in 20 mL 1,2-Dimethoxyethan zugetropft und anschließend das Kühlbad entfernt und die Reaktionslöung weitere 30min gerührt. Anschließend wurde für 2h am Rückfluß erhitzt, auf Raumtemperatur abgekühlt und mit wässr: Ammoniumchloridlösung versetzt und mit Essigester extrahiert (3x). Die vereinigten organischen Phasen wurden mit ges. Kochsalzlösung gewaschen, getrocknet und am Rotationsverdampfer eingeengt. Flashchromatographie (Cyclohexan/Essigester = 20:1 → 15:1 → 10:1) lieferte 514 mg (63%) 17(20)-Difluormethylen-3-hydroxy-2-methoxy-estra-1,3,5(10)-trien als amorphen Feststoff.
¹H-NMR (CDCl₃): δ = 0.92 (s, 3H; 18-CH₃), 3.86 (s, 3H; 2-OCH₃), 5.43 (s, 1H; 3-OH); 6.64, 6.78 (2 s, 2H; 1-H, 4-H) - ¹⁹F-NMR (CDCl₃): δ = - 91.1, - 96.4 (2d, *²J* = 68.9 Hz).

113 mg 17(20)-Difluormethylen-3-hydroxy-2-methoxy-estra-1,3,5(10)-trien wurden nach der allgemeinen Synthesevorschrift 1 (Base jedoch im Überschuss bzgl. Sulfamoylchlorid) zum Produkt umgesetzt und anschließende durch Flashchromatographie (Cyclohexan/Essigester = 3:1 → 2:1) gereinigt. Es wurden 101 mg (72%) 17(20)-Difluormethylen-2-methoxy-estra-1,3,5(10)-trien-3-yl sulfamat (8) als amorphes Pulver erhalten.
¹H-NMR (CDCl₃): δ = 0.93 (s, 3H; 18-CH₃), 3.88 (s, 3H; 2-OCH₃), 4.96 (s, 2H; NH₂), 6.92, 7.03 (2 s, 2H; 1-H, 4-H) - ¹⁹F-NMR (CDCl₃): δ = - 91.9, - 96.2 (2d, *²J* = 68.9 Hz).

### Beispiel 9

### 17β-Difluormethyl-2-methoxy-estra-1,3,5(10)-trien-3-yl sulfamat (9):

200 mg 17(20)-Difluormethylen-3-hydroxy-2-methoxy-estra-1,3,5(10)-trien wurden in 10 mL Essigester gelöst und dann mit 3 Tropfen Essigsäure und 70 mg Palladium auf Aktivkohle (10%-ig) versetzt. Die Hydrierung erfolgte unter Normaldruck über Nacht. Der Katalysator wurde abfiltriert und die Lösung am Rotationsverdampfer eingeengt und mehrmals mit Toluol coevaporiert. Flashchromatographie (Cyclohexan/Essigester = 10:1 → 5:1) lieferte 150 mg (74%) 17β-Difluormethyl-3-hydroxy-2-methoxy-estra-1,3,5(10)-trien als farblosen Feststoff.
¹H-NMR (CDCl₃): δ = 0.81 (s, 3H; 18-CH₃), 3.85 (s, 3H; 2-OCH₃), 5.42 (s, 1H; OH), 5.73 (td, 1H; CHF₂), 6.63, 6.77 (2 s, 2H; 1-H, 4-H) - ¹⁹F-NMR (CDCl₃): , δ = -113.5 (ddd, *²J_{F,F}* = 285.6 Hz, *²J_{F,H}* = 57.6 Hz, *³J_{F,H}* = 10.2 Hz), - 117.9 (ddd, *²J_{F,F}* = 293.0 Hz, *²J_{F,H}* = 56.5 Hz, *³J_{F,H}* = 12.8 Hz).

80 mg 17β-Difluormethyl-3-hydroxy-2-methoxy-estra-1,3,5(10)trien wurden nach der allgemeinen Synthese-vorschrift 1 zum Produkt umgesetzt und anschließend durch Flashchromatographie (Cyclohexan/Essigester = 4:1) gereinigt. Es wurden 82 mg (83%) 17β-Difluoromethyl-2-methoxy-estra-1,3,5(10)-trien-3-yl sulfamat (9) als farblose Kristalle erhalten.
¹H-NMR (CDCl₃): δ = 0.81 (s, 3H; 18-CH₃). 2.79-2.82 (m, 2H; 6-CH₂), 3.87 (s, 3H; 2-OCH₃), 4.95 (s, 2H; NH₂), 5.73 (td, 1H; CHF₂), 6.91, 7.03 (2 s, 2H; 1-H, 4-H) - ¹⁹F-NMR (CDCl₃):, δ = -113.6 (ddd, *²J_{F,F}* = 285.6 Hz, *²J_{F,H}* = 59.1, Hz, *³J_{F,H}* = 11.7 Hz), - 117.9 (ddd, *²J_{F,F}* = 285.6 Hz, *²J_{F,H}* = 56.1 Hz, *³J_{F,H}* = 12.8 Hz).

### Beispiel 10 (Vergleichsbeispiel)

### 17β-Carbaldehyd-2-methoxy-estra-1,3,5(10)-trien-3-yl sulfamat (10):

3.02 g 3-Hydroxy-2-methoxy-estra-1,3,5(10)-trien-17-on wurden mit 4.1 g Trimethylsulfonlumiodid in 30 mL abs. Dimethylformamid suspendiert. Bei 10°C wurde portionsweise 3.95 g Kalium-tert.-butylat zugegeben und anschließend langsam auf Raumtemperatur erwärmt. Nach 30min wurde auf Eiswasser gegossen und nach Neutralisation mit ges. Ammoniumchloridlösung mit Essigester extrahiert (3x). Die vereinigten organischen Phasen wurden mit Wasser und anschließend mit ges. Kochsalzlösung gewaschen, getrocknet und am Rotationsverdampfer eingeengt. Man erhielt 3-Hydroxy-2-methoxy-estra-1,3,5(10)-trien-17β-spiro-1',2'-oxiran in quantitativer Ausbeute (3.19 g) als farblose Kristalle.
¹H-NMR (CDCl₃): δ = 0.93 (s, 3H; 18-CH₃), 2.65, 2.96 (2 d, ²*J* = 4.9 Hz, 2H; Oxiran-CH₂), 3.85 (s, 3H; 2-OCH₃), 5.43 (s, 1H; 3-OH), 6.64, 6.77 (2 s, 2H; 1-H, 4-H).

3.19 g 3-Hydroxy-2-methoxy-estra-1,3,5(10)-trien-17β-spiro-1',2'-oxiran und 2 g Natriumazid wurden in 30 mL Ethylenglycol suspendiert und unter Argon auf 100°C erhitzt. Nach 1.5h wurde die Lösung abgekühlt, mit ges. Ammoniumchloridlösung versetzt und mit Dichlormethan extrahiert (3x, insg. 0.25 L). Die vereinigten organischen Phasen wurden mit Wasser gewaschen, getrocknet und am Rotationsverdampfer eingeengt. Nach Auskristallisation aus Essigester erhält man 17α-Azidomethyl-3,17β-Dihydroxy-2-methoxy-estra-1,3,5(10)-trien in quantitativer Ausbeute (3.61 g) als weißen Feststoff.
¹H-NMR (DMSO-D₆): δ = 0.82 (s, 3H; 18-CH₃), 3.09, 3.43 (2 d, ²*J* = 12.5 Hz, 2H; CH₂N₃), 6.45, 6.74 (2 s, 2H; 1-H, 4-H).

3.67 g 17α-Aidomethyl-3,17β-Dihydroxy-2-methoxy-estra-1,3,5(10)-trien wurden in 80 mL Dichlormethan suspendiert und unter Argon bei Raumtemperatur mit 3.7 g Triphenylphosphin versetzt. Nach 16h wurde wenig Wasser zugegeben und am Rotationsverdampfer eingeengt und mehrmals mit Toluol coevaporiert. Flashchromatographie der Rückstandes (Touol/Essigester = 1:0 → 20:1) lieferte 1.95 g (62%) einer α/β-Epimerenmischung, aus denen aus Aceton 1.0 g 17β-Carbaldehyd-2-methoxy-estra-1,3,5(10)-trien-3-ol als farblose Kristalle ausfiel.
¹H-NMR (CDCl₃): δ = 0.80 (s, 3H; 18-CH₃), 3.86 (s, 3H; 2-OCH₃), 5.07 (s, 2H; NH₂), 5.43 (s, 1H; OH), 6.64, 6.77 (2 s, 2H; 1-H, 4-H), 9.80 (s, 1H; CHO).

125 mg 17β-Carbaldehyd-2-methoxy-estra-1,3,5(10)-trien-3-ol wurden nach der allgemeinen Synthesevorschrift 1 zum Produkt umgesetzt und anschließend durch Flashchromatographie (Toluol/Essigester = 10:1 → 4:1) gereinigt. Es wurden 127 mg (81%) 17β-Carbaldehyd-2-methoxy-estra-1,3,5(10)-trien-3-yl sulfamat (**10**) als farbloser Schaum erhalten.
¹H-NMR (DMSO-d₆): δ = 0.71 (s, 3H; 18-CH₃), 3.76 (s, 3H; 2-OCH₃), 5.07 (s, 2H; NH₂), 6.97, 6.99 (2 s, 2H; 1-H, 4-H), 7.82 (s, 2H; NH₂), 9.75 (d, ³*J* = 1.6 Hz, 1H; CHO).

### Beispiel 11

### 17β-Hydroxymethyl-2-methoxy-estra-1,3,5(10)-trien-3-yl sulfamat (11):

89 mg 17β-Formyl-2-methoxy-estra-1,3,5(10)-trien-3-ol wurden in 2 mL Methanol und 6 mL Tetrahydrofuran gelöst und bei Raumtemperatur mit 83 mg Natriumborhydrid versetzt Nach 1h wurde mit wenig Essigsäure und Kieselgel versetzt und am Rotationsverdampfer eingeengt Flashchromatographie (Toluol/Essigester = 3:2) lieferte 75 mg (84%) 17β-Hydroxymethyl-2-methoxy-estra-1,3,5(10)-trien-3-yl sulfamat (11) als farblosen Schaum.
¹H-NMR (DMSO-d₆): δ = 0.63 (s, 3H; 18-CH₃), 3.47-3.52 (m, 1H; C*H*HOH), 3.76 (s, 3H; 2-OCH₃), 4.26 (d, ²*J* = 4.7 Hz, 1H; OH), 5.07 (s, 2H; NH₂), 6.97 (s, 2H; 1-H, 4-H), 7.81 (s, 2H; NH₂).

### Beispiel 12 (Vergleichsbeispiel)

### 2-Methoxy-6-oxo-18a-homoestra-1,3,5(10)-trien-3-yl sulfamat (12):

3-Acetoxy-2-methoxy-18a-homoestra-1,3,5(10)-trien wurde in 6-Position mit Chromtrioxid in Essigsäure bei 10°C in einer Ausbeute von 71% oxidiert und anschließend die Acetylgruppe durch Natriummethanolat in Methanol quantitativ abgespalten. Sulfamoylierung des erhaltenen Rückstandes nach der allgemeinen Synthesevorschrift 1 und anschließende Flashchromatographie (Toluol/Essigester = 5:1) lieferte 2-Methoxy-6-oxo-18a-homoestra-1,3,5(10)-trien-3-yl sulfamat (**12**) in 90% Ausbeute als farblosen Schaum.
¹H-NMR (CDCl₃): δ = 0.81 (t, *³J* = 7.4 Hz, 3H; 18-CH₃), 244-2.74 (m, 2H; 7-CH₂), 3.97 (s, 3H; 2-OCH₃), 5.37 (s, 2H; NH₂), 6.97, 7.95 (2 s, 2H; 1-H, 4-H).

### Beispiel 13 (Vergleichsbeispiel)

### 2-Methoxy-6-oximino-18a-homoestra-1,3,5(10)-trien-3-yl sulfamat (13):

61 mg 2-Methoxy-8-oxo-18a-homoestra-1,3,5(10)-trien-3-yl sulfamat (**12**) wurden mit 65 mg Hydroxylamin-hydrochlorid und 78 mg Natriumhydrogencarbonat in 3 mL Methanol für 4h auf 60°C erhitzt. Anschließend wurde auf Raumtemperatur abgekühlt, mit Wasser versetzt und mit Dichlormethan extrahiert (3x). Die vereinigten organischen Phasen wurden getrocknet und am Rotationsverdampfer eingeengt. Flashchromatographie (Toluol/Aceton = 9:1 → 5:1) lieferte 38 mg (60%) 2-Methoxy-6-oximino-18a-homoestra-1,3,5(10)-trien-3-yl sulfamat (**13**) als amorphen Feststoff.
¹H-NMR (Aceton): δ = 0.82 (t, *³J* = 7.4 Hz, 3H; 18-CH₃), 3.18 (dd, *²J* = 18.0, *³J* = 4.3 Hz, 1H; 7-CH), 3.90 (s, 3H; 2-OCH₃), 6.96 (br s, 2H; NH₂), 7.04, 7.89 (2 s, 2H; 1-H, 4-H), 10.07 (s, 1H; OH).

### Beispiel 14 (Vergleichsbeispiel)

### 2-Methoxy-6-(O-methyloximino)-18a-homoestra-1,3,5(10)-trien-3-yl sulfamat (14):

74 mg 2-Methoxy-6-oxo-18a-homoestra-1,3,5(10)-trien-3-yl sulfamat (**12**) wurden mit 158 mg O-Methylhydroxylamin-hydrochlorid und 157 mg Natriumhydrogencarbonat in 3 mL Methanol für 3h auf 70°C erhitzt. Anschließend wurde auf Raumtemperatur abgekühlt, mit Wasser versetzt und mit Dichlormethan extrahiert (3x). Die vereinigten organischen Phasen wurden getrocknet und am Rotationsverdampfer eingeengt. Flashchromatographie (Toluol/Aceton = 14:1 → 9:1) lieferte 59 mg (74%) 2-Methoxy-6-(O-methyloximino)-18a-homoestra-1,3,5(10)-trien-3-yl sulfamat (**14**) als grauen Schaum.
¹H-NMR (CDCl₃): δ = 0.79 (t, *³J* = 7.4 Hz, 3H; 18-CH₃), 3.18 (dd, *²J* = 18.2, *³J* = 4.5 Hz, 1H; 7-CH), 3.91 (s, 3H; NOCH₃), 3.95 (s, 3H; 2-OCH₃), 5.21 (s, 2H; NH₂), 6.90, 7.89 (2 s, 2H; 1-H, 4-H).

### Beispiel 15 (Vergleichsbeispiel)

### 6α-Hydroxy-2-methoxy-18a-homoestra-1,3,5(10)-trien-3-yl sulfamat (15):

2-Methoxy-6-oxo-18a-homoestra-1,3,5(10)-trien-3-yl sulfamat (**12**) wurde in Methanol gelöst und im Eisbad mit einem Überschuß an Natriumborhydrid versetzt. Das Eisbad wurde entfernt und nach 2h wurde mit Aceton versetzt und am Rotationsverdampfer eingeengt Der Rückstand wurde mit wässr. Ammoniumchloridlösung versetzt und mit Dichlormethan extrahiert (3x). Die vereinigten organischen Phasen wurden getrocknet und am Rotationsverdampfer eingeengt. Flashchromatographie (Toluol/Essigester = 5:1 → 3:1) lieferte rund 50% 6α-Hydroxy-2-methoxy-18a-homoestra-1,3,5(10)-trien-3-yl sulfamat (**15**) als farblose Kristalle.
¹H-NMR (CDCl₃): δ = 0.78 (t, *³J* = 7.4 Hz, 3H; 18-CH₃), 3.87 (s, 3H; 2-OCH₃), 4.77 (dd, 1H; 6β-H), 5.32 (s, 2H; NH₂), 6.88, 7.44 (2 s, 2H; 1-H, 4-H).

### Beispiel 16 (Vergleichsbeispiel)

### 17α-Fluor-2-methoxy-estra-1,3,5(10)-trien-3-yl sulfamat (16):

215 mg 2-Methoxy-estra-1,3,5(10)-trien-3,17β-diol wurden in 20 mL abs. Dichlormethan gelöst und auf -35°C gekühlt. Anschließend wurde 280 µL Diethylaminoschwefeltrifluorid zugegeben und das Kältebad entfernt. Nach 1h wurde auf wässr. Natriumhydrogencarbonatisg. gegossen und mit Dichlormethan extrahiert (3x). Die vereinigten organischen Phasen wurden getrocknet und am Rotationsverdampfer eingeengt. Flashchromatographie (Cyclohexan/Essigester = 10:1) lieferte 49% Rohprodukt, welches mittels HPLC (Chiracel OD-H 250x4.6 mm; n-Heptan/2-Propanol = 95/5) gereinigt wurde. Man erhielt 46 mg (21%) 17α-Fluor-3-hydroxy-2-methoxy-estra-1,3,5(10)-trien als farblose Kristalle.
¹H-NMR (CDCl₃): δ = 0.70 (d, 3H; 18-CH₃), 3.85 (s, 3H; 2-OCH₃), 4.57 (dd, *J_{HF}* = 55.3, *J_{HH}* = 5.3 Hz, 1H; 17β-H), 5.46 (s, 1H; OH), 6.64, 6.80 (2 s, 2H; 1-H, 4-H) - ¹⁹F-NMR (CDCl₃): δ = - 177.25 (ddd, *²J* = 70.4, *³J* = 34.6 und 21.5 Hz).

34 mg 17α-Fluor-3-hydroxy-2-methoxy-estra-1,3,5(10)-trien wurden nach der allgemeinen Synthesevorschrift 1 zum Produkt umgesetzt und anschließend durch Flashchromatographie (Toluol/Essigester = 12:1 → 5:1) gereinigt. Es wurden 42 mg (98%) 17α-Fluor-2-methoxy-estra-1,3,5(10)-trien-3-yl sulfamat (**16**) als amorpher Feststoff erhalten.
¹H-NMR (CDCl₃): δ = 0.71 (d, *J* = 1.6 Hz, 3H; 18-CH₃), 2.79-2.82 (m, 2H; 6-CH₂), 3.88 (s, 3H; 2-OCH₃), 4.58 (dd, *J_{NF}* = 55.5, *J_{HN}* = 5.1 Hz, 1H; 17β-H), 5.29 (s, 2H; NH₂), 6.94, 7.04 (2 s, 2H; 1-H, 4-H) - ¹⁹F-NMR (CDCl₃): δ = -177.38 (ddd, *²J* = 70.4, *³J* = 34.6 und 19.9 Hz).

### Beispiel 17 (Vergleichsbeispiel)

### 2-Methoxy-(E)-17-(oximino)-estra-1,3,5(10)-trien-3-yl sulfamat (17):

Eine Suspension von 570 mg 2-Methoxy-17-oxo-estra-1,3,5(10)-trien-3-yl sulfamat, 365 mg Hydroxylaminhydrochlorid und 441 mg Natriumhydrogencarbonat in 8 mL Methanol wurde unter Rückfluß eine Stunde gerührt. Dann wurde mit je 30 mL Wasser und Essigester versetzt. Nach der Phasentrennung wurde die wässrige Phase noch 2 mal mit je 15 mL Essigester extrahiert. Die vereinigten organischen Phasen wurden zunächst mit 15 mL 0.5N HCI und dann mit ges. NaCl-Lösung gewaschen, über Natriumsulfat getrocknet und am Rotationsverdampfer eingeengt. Das Rohprodukt wurde flashchromatographlsch gereinigt (Toluol/Essigester 3:1). Man erhielt 444 mg (73%) 2-Methoxy-(*E*)-17-(oximino)-estra-1,3,5(10)trien-3-yl sulfamat (**17**) als farblose Kristalle.
¹H-NMR (DMSO-d₆): δ = 0.87 (s, 3H; 13-CH₃), 3.76 (s, 3H; 2-OCH₃), 6.98, 6.99 (2 s, 2H; 1-H, 4-H), 7.82 (s, 2H; NH₂), 10.09 (s, 1H; N-OH)

## Patentansprüche

1. Verwendung der 2-substituierten Estra-1,3,5(10)-trien-3-yl-sulfamate der allgemeinen Formel I zur Herstellung eines Medikaments für die Behandlung von Tumorerkrankungen auf die die Inhibierung der Tubulinpolymerisation einen positiven Einfluß hat 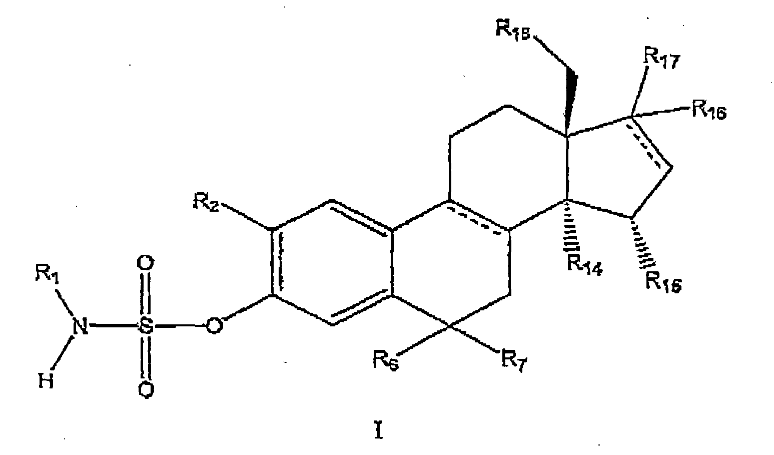 worin die Reste R¹ und R², R⁶ und R⁷, R¹⁴ und R¹⁵, R¹⁶ und R¹⁷ sowie R¹⁸ die folgenden Bedeutungen haben:
R¹ = Wasserstoff, C₁₋₅-Alkyl oder C₁₋₅-Acyl,
R² = C₁₋₅-Alkoxy, C₁₋₅-Alkyl oder ein Rest -O-CₙFₘHₒ, wobei n = 1, 2, 3, 4, 5 oder 6, in > 1 und m+o = 2n+1,
wenn R² Alkyl darstellt, R¹⁷ C₁₋₅-Alkoxy sein kann,
R⁶ = Wasserstoff,
R⁷ = Wasserstoff, Hydroxy, Amino, Acylamino, wobei
wenn R⁶ und R⁷ nicht Wasserstoff sind, R¹⁷ C₁₋₅-Alkoxy sein kann, oder
R⁶ und R⁷ zusammen Sauerstoff, Oxim oder O(C₁₋₅-Alkyl)-Oxim darstellen,
R¹⁴ und R¹⁵ in jedem Fall Wasserstoff bedeuten können oder zusammen eine Methylengruppe oder eine zusätzliche Bindung bedeuten,
R¹⁶ Wasserstoff ist,
R¹⁷ eine Gruppe CHXY ist, in der X für ein Wasserstoffatom, Fluor, eine Hydroxygruppe oder einen C₁₋₄-Alkylrest steht, und Y für ein Wasserstoffatom oder Fluor steht, wobei wenn X Hydroxy ist, Y nur Wasserstoff sein kann,
R¹⁸ ein Wasserstoffatom oder eine Methylgruppe ist,
wobei wenn R¹⁸ eine Methylgruppe ist, R¹⁷ Sulfamat SO₃NHR¹ sein kann,
wobei im B- und D-Ring des Steroidgerüsts die gestrichelten Linien bis zu zwei Doppelbindungen bedeuten können,
sowie ihre pharmazeutisch verträglichen Salze.

2. Verwendung der 2-substituierten Estra-1,3,5(10)-trien-3-yl-sulfamate nach Anspruch 1, wobei R¹ Wasserstoff oder C₁₋₅-Acyl darstellt.

3. Verwendung der 2-substituierten Estra-1,3,5(10)-trien-3-yl-sulfamate nach Anspruch 1, wobei R² Methyl, Ethyl, Methoxy, Ethoxy oder 2,2,2-Trifluorethoxy darstellt.

4. Verwendung der 2-substituierten Estra-1,3,5(10)-trien-3-yl-sulfamate nach Anspruch 1, wobei wenn R² ein Alkyl ist, R¹⁷ C₁₋₅-Alkoxy darstellt.

5. Verwendung der 2-substituierten Estra-1,3,5(10)-trien-3-yl-sulfamate nach Anspruch 1, wobei R⁶ und R⁷ in jedem Fall Wasserstoff sind oder zusammen ein Oxim darstellen.

6. Verwendung der 2-substituierten Estra-1,3,5(10)-trien-3-yl-sulfamate nach Anspruch 1, wobei R¹⁴ und R¹⁵ in jedem Fall Wasserstoff sind oder zusammen eine Methylengruppe darstellen.

7. Verwendung der 2-substituierten Estra-1,3,5(10)-trien-3-yl-sulfamate nach Anspruch 1, wobei R¹⁷ Methyl, Difluormethyl, oder Hydroxylmethyl darstellt.

8. Verwendung der 2-substituierten Estra-1,3,5(10)-trien-3-yl-sulfamate nach Anspruch 1, wobei R¹⁸ Wasserstoff oder Methyl darstellt.

9. Verwendung der 2-substituierten Estra-1,3,5(10)-trien-3-yl-sulfamate nach Anspruch 1, wobei die Verbindung ausgewählt ist aus der Verbindungsgruppe, bestehend aus 2-Ethyl-17β-methoxy-estra-1,3,5(10)-trien-3-yl-sulfamat, 2-Methoxy-17β-methyl-estra-1,3,5(10)-trien-3-yl-sulfamat, 17β-Difluormethyl-2-methoxy-estra-1,3,5(10)-trien-3-yl-sulfamat, 17β-Hydroxymethyl-2-methoxy-estra-1,3,5(10)-trien-3-yl-sulfamat, 2,17β-Dimethoxy-6-oximino-18a-homoestra-1,3,5(10)-trien-3-yl-sulfamat, 17β-Ethoxy-2-methoxy-6-oximino-18a-homoestra-1,3,5(10)-trien-3-yl-sulfamat,

10. Verwendung der 2-substituierten Estra-1,3,5(10)-trien-3-yl-sulfamate nach einem der Ansprüche 1 bis 9 zur Herstellung eines pharmazeutischen Mittels für die Behandlung von Tumorerkrankungen der männlichen und weiblichen Keimdrüsen, der männlichen und weiblichen Sexualorgane, einschließlich der Brustdrüsen.

11. Verwendung der 2-substituierten Estra-1,3,5(10)-trien-3-yl-sulfamate nach einem der Ansprüche 1 bis 10 zur Herstellung eines pharmazeutischen Mittels für die Behandlung Brustkrebs.

12. Verwendung der 2-substituierten Estra-1,3,5(10)-trien-3-yl-sulfamate nach einem der Ansprüche 1 bis 11 zur Herstellung eines pharmazeutischen Mittels für die Behandlung von Prostatakrebs.

13. Verwendung der 2-substituierten Estra-1,3,5(10)-trien-3-yl-sulfamate nach Anspruch 12 zur Herstellung eines pharmazeutischen Mittels für die Behandlung von Prostatakrebs, wobei mindestens ein zusätzlicher Wirkstoff verwendet wird.

14. 2-substituierte Estra-1,3,5(10)-trien-3-yl-sulfamate der allgemeinen Formel I zur Behandlung von Tumorerkrankungen, auf die die Inhibierung der Tubulinpolymerisation einen positiven Einfluß hat 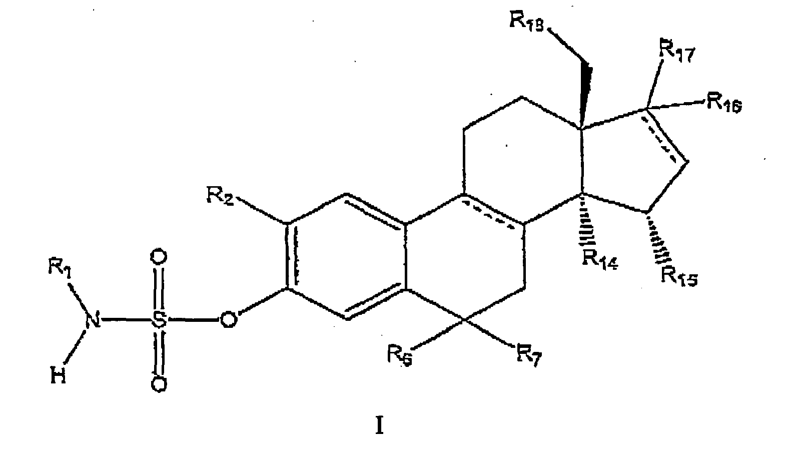 worin die Reste R¹ und R², R⁶ und R⁷, R¹⁴ und R¹⁵, R¹⁶ und R¹⁷ sowie R¹⁸ die folgenden Bedeutungen haben:
R¹ = Wasserstoff, C₁₋₅-Alkyl oder C₁₋₅-Acyl,
R² = C₁₋₅-Alkoxy, C₁₋₅-Alkyl oder ein Rest -O-CₙFₘHₒ, wobei n = 1, 2, 3, 4, 5 oder 6, m > 1 und m+o = 2n+1,
wenn R² Alkyl darstellt, R¹⁷ C₁₋₅-Alkoxy sein kann,
R⁶ = Wasserstoff,
R⁷ = Wasserstoff, Hydroxy, Amino, Acylamino, wobei
wenn R⁶ und R⁷ nicht Wasserstoff, sind, R¹⁷ C₁₋₅-Alkoxy sein kann, oder
R⁶ und R⁷ zusammen Sauerstoff, Oxim oder O(C₁₋₅-Alkyl)-Oxim darstellen,
R¹⁴ und R¹⁵ in jedem Fall Wasserstoff bedeuten können oder zusammen eine Methylengruppe oder eine zusätzliche Bindung bedeuten,
R¹⁶ Wasserstoff ist,
R¹⁷ eine Gruppe CHXY ist, in der X für ein Wasserstoffatom, Fluor, eine Hydroxygruppe oder einen C₁₋₄-Alkylrest steht, und Y für ein Wasserstoffatom oder Fluor steht, wobei
wenn X Hydroxy ist, Y nur Wasserstoff sein kann,
R¹⁸ ein Wasserstoffatom oder eine Methylgruppe ist,
wobei wenn R¹⁸ eine Methylgruppe ist, R¹⁷ Sulfamat SO₃NHR¹ sein kann,
wobei im B- und D-Ring des Steroidgerüsts die gestrichelten Linien bis zu zwei Doppelbindungen bedeuten können,
sowie ihre pharmazeutisch verträglichen Salze.

15. 2-substituierte Estra-1,3,5(10)-trien-3-yl-sulfamate nach Anspruch 14, wobei R¹ Wasserstoff oder C₁₋₅-Acyl darstellt.

16. 2-substituierte Estra-1,3,5(10)-trien-3-yl-sulfamate nach Anspruch 14, wobei R² Methyl; Ethyl, Methoxy, Ethoxy oder 2,2,2-Trifluorethoxy darstellt.

17. 2-substituierte Estra-1,3,5(10)-trien-3-yl-sulfamate nach Anspruch 14, wobei wenn R² ein Alkyl ist, R¹⁷ C₁₋₅-Alkoxy darstellt.

18. 2-substituierte Estra-1,3,5(10)-trien-3-yl-sulfamate nach Anspruch 14, wobei R⁶ und R⁷ in jedem Fall Wasserstoff sind oder zusammen ein Oxim darstellen.

19. 2-substituierte Estra-1,3,5(10)-trien-3-yl-sulfamate nach Anspruch 14, wobei R¹⁴ und R¹⁵ in jedem Fall Wasserstoff sind oder zusammen eine Methylengruppe darstellen.

20. 2-substituierte Estra-1,3,5(10)-trien-3-yl-sulfamate nach Anspruch 14, wobei R¹⁷ Methyl, Difluormethyl, oder Hydroxylmethyl darstellt.

21. 2-substituierte Estra-1,3,5(10)-trien-3-yl-sulfamate nach Anspruch 14, wobei R¹⁸ Wasserstoff oder Methyl darstellt.

22. 2-substituierte Estra-1,3,5(10)-trien-3-yl-sulfamate nach Anspruch 14, wobei die Verbindung ausgewählt ist aus der Verbindungsgruppe, bestehend aus 2-Ethyl-17β-methoxy-estra-1,3,5(10)-trien-3-yl-sulfamat, 2-Methoxy-17β-methyl-estra-1,3,5(10)-trien-3-yl-sulfamat, 17β-Difluormethyl-2-methoxy-estra-1,3,5(10)-trien-3-yl-sulfamat, 17β-Hydroxymethyl-2-methoxy-estra-1,3,5(10)-trien-3-yl-sulfamat, 2,17β-Dimethoxy-6-oximino-18a-homoestra-1,3,5(10)-trien-3-yl-sulfamat, 17β-Ethoxy-2-methoxy-6-oximino-18a-homoestra-1,3,5(10)-trien-3-yl-sulfamat.

23. 2-substituierte Estra-1,3,5(10)-trien-3-yl-sulfamate nach einem der Ansprüche 14 bis 22 für die Herstellung eines pharmazeutischen Mittels für die Behandlung von Tumorerkrankungen der männlichen und weiblichen Keimdrüsen, der männlichen und weiblichen Sexualorgane, einschließlich der Brustdrüsen.

24. 2-substituierte Estra-1,3,5(10)-trien-3-yl-sulfamate nach einem der Ansprüche 14 bis 23 für die Herstellung eines pharmazeutischen Mittels für die Behandlung Brustkrebs.

25. 2-substituierte Estra-1,3,5(10)-trien-3-yl-sulfamate nach einem der Ansprüche 14 bis 24 für die Herstellung eines pharmazeutischen Mittels für die Behandlung von Prostatakrebs.

26. 2-substituierte Estra-1,3,5(10)-trien-3-yl-sulfamate nach Anspruch 25 für die Herstellung eines pharmazeutischen Mittels für die Behandlung von Prostatakrebs, wobei mindestens ein zusätzlicher Wirkstoff verwendet wird.

## Claims

1. Use of the 2-substituted estra-1,3,5(10)-triene-3-yl-sulphamates of general formula I for the preparation of a medicament for the treatment of tumor diseases on which the inhibition of tubulin polymerization has a positive influence 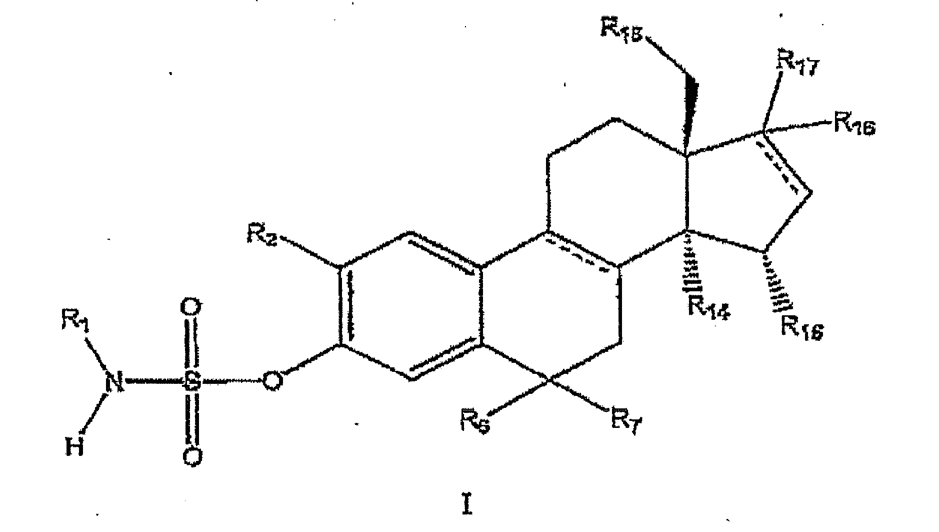 in which the radicals R¹ and R², R⁶ and R⁷, R¹⁴ and R¹⁵, R¹⁶ and R¹⁷ as well as R¹⁸ have the following meanings:
R¹ = hydrogen, C₁₋₅ alkyl or C₁₋₅ acyl,
R² = C₁₋₅ alkoxy, C₁₋₅ alkyl or a radical -O-CₙFₘHₒ, wherein n = 1,2,3,4,5 or 6, m > 1 and m+o = 2n + 1,
if R² represents alkyl, R¹⁷ can be C₁₋₅ alkoxy,
R⁶ = hydrogen,
R⁷ = hydrogen, hydroxy, amino, acylamino, wherein
if R⁶ and R⁷ are not hydrogen, R¹⁷ can be C₁₋₅ alkoxy, or
R⁶ and R⁷ together represent oxygen, oxime or O(C₁₋₅ alkyl)-oxime,
R¹⁴ and R¹⁵ can in every case mean hydrogen or together mean a methylene group or an additional bond,
R¹⁶ is hydrogen,
R¹⁷ is a group CHXY in which X stands for a hydrogen atom, fluorine, a hydroxy group or a C₁₋₄ alkyl radical, and Y stands for a hydrogen atom or fluorine, wherein if X is hydroxy, Y can only be hydrogen,
R¹⁸ is a hydrogen atom or a methyl group,
wherein if R¹⁸ is a methyl group, R¹⁷ can be sulphamate SO₃NHR¹,
wherein the dotted lines in the B and the D ring of the steroid skeleton can mean up to two double bonds,
as well as their pharmaceutically compatible salts.

2. Use of the 2-substituted estra-1,3,5(10)-triene-3-yl-sulphamates according to claim 1, wherein R¹ represents hydrogen or C₁₋₅ acyl.

3. Use of the 2-substituted estra-1,3,5(10)-triene-3-yl-sulphamates according to claim 1, wherein R² represents methyl, ethyl, methoxy, ethoxy or 2,2,2-trifluorethoxy.

4. Use of the 2-substituted estra-1,3,5(10)-triene-3-yl-sulphamates according to claim 1, wherein if R² is an alkyl, R¹⁷ represents C₁₋₅ alkoxy.

5. Use of the 2-substituted estra-1,3,5(10)-triene-3-yl-sulphamates according to claim 1, wherein R⁶ and R⁷ are in every case hydrogen or together represent an oxime.

6. Use of the 2-substituted estra-1,3,5(10)-triene-3-yl-sulphamates according to claim 1, wherein R¹⁴ and R¹⁵ are in every case hydrogen or together represent a methylene group.

7. Use of the 2-substituted estra-1,3,5(10)-triene-3-yl-sulphamates according to claim 1, wherein R¹⁷ represents methyl, difluoromethyl or hydroxylmethyl.

8. Use of the 2-substituted estra-1,3,5(10)-triene-3-yl-sulphamates according to claim 1, wherein R¹⁸ represents hydrogen or methyl.

9. Use of the 2-substituted estra-1,3,5(10)-triene-3-yl-sulphamates according to claim 1, wherein the compound is selected from the group of compounds consisting of
2-ethyl-17β-methoxy-estra-1,3,5(10)-triene-3-yl-sulphamate,
2-methoxy-17β-methoxy-estra-1,3,5(10)-triene-3-yl-sulphamate,
17β-difluoromethyl-2-methoxy-estra-1,3,5(10)-triene-3-yl-sulphamate,
17β-hydroxymethyl-2-methoxy-estra-1,3,5(10)-triene-3-yl-sulphamate,
2,17β-dimethoxy-6-oximino-18a-homoestra-1,3,5(10)-triene-3-yl-sulphamate,
17β-ethoxy-2-methoxy-6-oximino-18a-homoestra-1,3,5(10)-triene-3-yl-sulphamate.

10. Use of the 2-substituted estra-1,3,5(10)-triene-3-yl-sulphamates according to one of claims 1 to 9 for the preparation of a pharmaceutical agent for the treatment of tumor diseases of the male and female gonads, the male and female sexual organs, including the mammary glands.

11. Use of the 2-substituted estra-1,3,5(10)-triene-3-yl-sulphamates according to one of claims 1 to 10 for the preparation of a pharmaceutical agent for the treatment of breast cancer.

12. Use of the 2-substituted estra-1,3,5(10)-triene-3-yl-sulphamates according to one of claims 1 to 11 for the preparation of a pharmaceutical agent for the treatment of prostate cancer.

13. Use of the 2-substituted estra-1,3,5(10)-triene-3-yl-sulphamates according to claim 12 for the preparation of a pharmaceutical agent for the treatment of prostate cancer, wherein at least one additional active ingredient is used.

14. 2-substituted estra-1,3,5(10)-triene-3-yl-sulphamates of the general Formula I for the treatment of tumor diseases on which the inhibition of tubulin polymerization has a positive influence 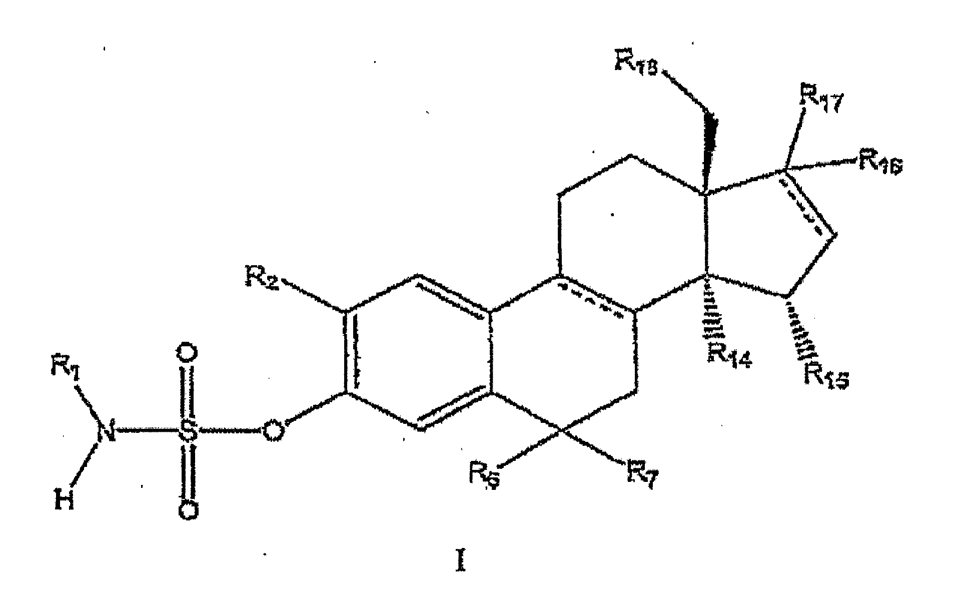 in which the radicals R¹ and R², R⁶ and R⁷, R¹⁴ and R¹⁵, R¹⁶ and R¹⁷ as well as R¹⁸ have the following meanings:
R¹ = hydrogen, C₁₋₅ alkyl or C₁₋₅ acyl,
R² = C₁₋₅ alkoxy, C₁₋₅ alkyl or a radical -O-CₙFₘHₒ, wherein n = 1,2,3,4,5 or 6, m > 1 and m+o = 2n + 1,
if R² represents alkyl, R¹⁷ can be C₁₋₅ alkoxy,
R⁶ = hydrogen,
R⁷ = hydrogen, hydroxy, amino, acylamino, wherein
if R⁶ and R⁷ are not hydrogen, R¹⁷ can be C₁₋₅ alkoxy, or
R⁶ and R⁷ together represent oxygen, oxime or O(C₁₋₅ alkyl)-oxime,
R¹⁴ and R¹⁵ can in every case mean hydrogen or together mean a methylene group or an additional bond,
R¹⁶ is hydrogen,
R¹⁷ is a group CHXY in which X stands for a hydrogen atom, fluorine, a hydroxy group or a C₁₋₄ alkyl radical, and Y stands for a hydrogen atom or fluorine, wherein if X is hydroxy, Y can only be hydrogen,
R¹⁸ is a hydrogen atom or a methyl group,
wherein if R¹⁸ is a methyl group, R¹⁷ can be sulphamate SO₃NHR¹,
wherein the dotted lines in the B and the D ring of the steroid skeleton can mean up to two double bonds,
as well as their pharmaceutically compatible salts.

15. 2-substituted estra-1,3,5(10)-triene-3-yl-sulphamates according to claim 14, wherein R¹ represents hydrogen or C₁₋₅ acyl.

16. 2-substituted estra-1,3,5(10)-triene-3-yl-sulphamates according to claim 14, wherein R² represents methyl, ethyl, methoxy, ethoxy or 2,2,2-trifluoroethoxy.

17. 2-substituted estra-1,3,5(10)-triene-3-yl-sulphamates according to claim 14, wherein if R² is an alkyl, R¹⁷ represents C₁₋₅ alkoxy.

18. 2-substituted estra-1,3,5(10)-triene-3-yl-sulphamates according to claim 14, wherein R⁶ and R⁷ are in every case hydrogen or together represent an oxime.

19. 2-substituted estra-1,3,5(10)-triene-3-yl-sulphamates according to claim 14, wherein R¹⁴ and R¹⁵ are in every case hydrogen or together represent a methylene group.

20. 2-substituted estra-1,3,5(10)-triene-3-yl-sulphamates according to claim 14, wherein R¹⁷ represents methyl, difluoromethyl or hydroxylmethyl.

21. 2-substituted estra-1,3,5(10)-triene-3-yl-sulphamates according to claim 14, wherein R¹⁸ represents hydrogen or methyl.

22. 2-substituted estra-1,3,5(10)-triene-3-yl-sulphamates according to claim 14, wherein the compound is selected from the group of compounds consisting of 2-ethyl-17β-methoxy-estra-1,3,5(10)-triene-3-yl-sulphamate, 2-methoxy-17β-methoxy-estra-1,3,5(10)-triene-3-yl-sulphamate, 17β-difluoromethyl-2-methoxy-estra-1,3,5(10)-triene-3-yl-sulphamate, 17β-hydroxymethyl-2-methoxy-estra-1,3,5(10)-triene-3-yl-sulphamate, 2,17β-dimethoxy-6-oximino-18a-homoestra-1,3,5(10)-triene-3-yl-sulphamate, 17β-ethoxy-2-methoxy-6-oximino-18a-homoestra-1,3,5(10)-triene-3-yl-sulphamate.

23. 2-substituted estra-1,3,5(10)-triene-3-yl-sulphamates according to one of claims 14 to 22 for the preparation of a pharmaceutical agent for the treatment of tumor diseases of the male and female gonads, the male and female sexual organs, including the mammary glands.

24. 2-substituted estra-1,3,5(10)-triene-3-yl-sulphamates according to one of claims 14 to 23 for the preparation of a pharmaceutical agent for the treatment of breast cancer.

25. 2-substituted estra-1,3,5(10)-triene-3-yl-sulphamates according to one of claims 14 to 24 for the preparation of a pharmaceutical agent for the treatment of prostate cancer.

26. 2-substituted estra-1,3,5(10)-triene-3-yl-sulphamates according to claim 25 for the preparation of a pharmaceutical agent for the treatment of prostate cancer, wherein at least one additional active ingredient is used.

## Revendications

1. Utilisation des estra-1,3,5(10)-trién-3-yl-sulfamates 2-substitués de formule générale 1 pour la production d'un médicament pour le traitement des maladies tumorales sur lesquelles l'inhibition de la polymérisation de la tubuline a une influence positive 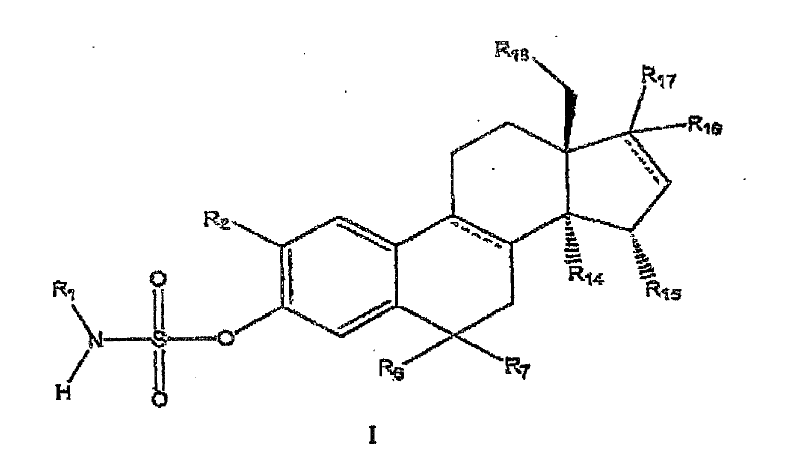 où les groupements R¹ et R², R⁶ et R⁷, R¹⁴ et R¹⁵, R¹⁶ et R¹⁷ ainsi que R¹⁸ ont les significations suivantes :
R¹ = hydrogène, C₁₋₅-alkyle ou C₁₋₅-acyle,
R² = C₁₋₅-alcoxy, C₁₋₅-alkyle ou un groupement -O-CₙFₘHₒ, où n = 1, 2, 3, 4, 5 ou 6, m > 1 et m + o = 2n + 1,
quand R² représente alkyle, R¹⁷ peut être C₁₋₅-alcoxy,
R⁶ = hydrogène,
R⁷ = hydrogène, hydroxy, amino, acylamino, où
quand R⁶ et R⁷ ne sont pas l'hydrogène, R¹⁷ peut être C₁₋₅-alcoxy, ou
R⁶ et R⁷ représentent ensemble l'oxygène, une oxime ou une O(C₁₋₅-alkyl)-oxime,
R¹⁴ et R¹⁵ peuvent signifier dans chaque cas l'hydrogène ou signifient ensemble un groupe méthylène ou une liaison supplémentaire,
R¹⁶ est l'hydrogène,
R¹⁷ est un groupe CHXY dans lequel X représente un atome d'hydrogène, le fluor,
un groupe hydroxy ou un groupement C₁₋₄-alkyle, et Y représente un atome d'hydrogène ou le fluor, où
quand X est hydroxy, Y ne peut être que l'hydrogène,
R¹⁸ est un atome d'hydrogène ou un groupe méthyle,
où quand R¹⁸ est un groupe méthyle, R¹⁷ peut être un sulfamate SO₃NHR¹,
où dans les cycles B et D du squelette stéroïde, les traits en pointillés peuvent signifier jusqu'à deux doubles liaisons,
ainsi que leurs sels pharmaceutiquement acceptables.

2. Utilisation des estra-1,3,5(10)-trién-3-yl-sulfamates 2-substitués selon la revendication 1 où R¹ représente l'hydrogène ou C₁₋₅-acyle.

3. Utilisation des estra-1,3,5(10)-trién-3-yl-sulfamates 2-substitués selon la revendication 1 où R² représente méthyle, éthyle, méthoxy, éthoxy ou 2,2,2-trifluoroéthoxy.

4. Utilisation des estra-1,3,5(10)-trién-3-yl-sulfamates 2-substitués selon la revendication 1 où, quand R² est un alkyle, R¹⁷ représente C₁₋₅-alcoxy.

5. Utilisation des estra-1,3,5(10)-trién-3-yl-sulfamates 2-substitués selon la revendication 1 où R⁶ et R⁷ sont dans chaque cas l'hydrogène ou représentent ensemble une oxime.

6. Utilisation des estra-1,3,5(10)-trién-3-yl-sulfamates 2-substitués selon la revendication 1 où R¹⁴ et R¹⁵ sont dans chaque cas l'hydrogène ou représentent ensemble un groupe méthylène.

7. Utilisation des estra-1,3,5(10)-trién-3-yl-sulfamates 2-substitués selon la revendication 1 où R¹⁷ représente méthyle, difluorométhyle ou hydroxyméthyle.

8. Utilisation des estra-1,3,5(10)-trién-3-yl-sulfamates 2-substitués selon la revendication 1 où R¹⁸ représente l'hydrogène ou méthyle.

9. Utilisation des estra-1,3,5(10)-trién-3-yl-sulfamates 2-substitués selon la revendication 1 où le composé est choisi dans le groupe de composés consistant en
2-éthyl-17β-méthoxy-estra-1,3,5(10)-trién-3-yl-sulfamate,
2-méthoxy-17β-méthyl-estra-1,3,5(10)-trién-3-yl-sulfamate,
17β-difluorométhyl-2-méthoxy-estra-1,3,5(10)-trién-3-yl-sulfamate,
17β-hydroxyméthyl-2-méthoxy-estra-1,3,5(10)-trién-3-yl-sulfamate,
2,17β-diméthoxy-6-oximino-18a-homoeslra-1,3,5(10)-trién-3-yl-sulfamate,
17β-éthoxy-2-méthoxy-6-oximino-18a-homoestra-1,3,5(10)-trién-3-yl-sulfamate,

10. Utilisation des estra-1,3,5(10)-trién-3-yl-sulfamates 2-substitués selon l'une des revendications 1 à 9 pour la production d'un agent pharmaceutique pour le traitement des maladies tumorales des glandes génitales masculines et féminines, des organes sexuels masculins et féminins, y compris des glandes mammaires.

11. Utilisation des estra-1,3,5(10)-triën-3-yl-sulfamates 2-substitués selon l'une des revendications 1 à 10 pour la production d'un agent pharmaceutique pour le traitement du cancer du sein.

12. Utilisation des estra-1,3,5(10)-trién-3-yl-sulfamates 2-substitués selon l'une des revendications 1 à 11 pour la production d'un agent pharmaceutique pour le traitement du cancer de la prostate.

13. Utilisation des estra-1,3,5(10)-trïén-3-yl-sulfamates 2-substitués selon la revendication 12 pour la production d'un agent pharmaceutique pour le traitement du cancer de la prostate, où au moins un principe actif supplémentaire est utilisé.

14. Estra-1,3,5(10)-trién-3-yl-sulfamates 2-substitués de formule générale 1 pour le traitement des maladies tumorales sur lesquelles l'inhibition de la polymérisation de la tubuline a une influence positive 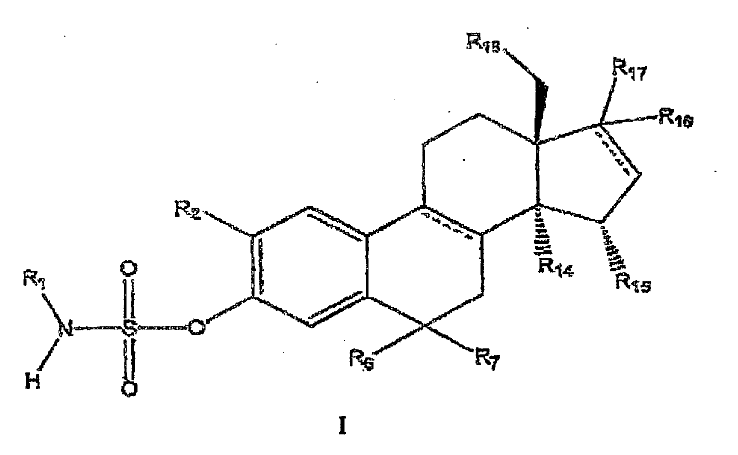 où les groupements R¹ et R², R⁶ et R⁷, R¹⁴ et R¹⁵, R¹⁶ et R¹⁷ ainsi que R¹⁸ ont les significations suivantes :
R¹ = hydrogène, C₁₋₅-alkyle ou C₁₋₅-acyle,
R² = C₁₋₅-alcoxy, C₁₋₅-alkyle ou un groupement -O-CₙFₘHₒ, où n = 1, 2, 3, 4, 5 ou 6, m > 1 et m+o = 2n + 1,
quand R² représente alkyle, R¹⁷ peut être C₁₋₅-alcoxy,
R⁶ = hydrogène,
R⁷ = hydrogène, hydroxy, amino, acylamino, où
quand R⁶ et R⁷ ne sont pas l'hydrogène, R¹⁷ peut être C₁₋₅-alcoxy, ou
R⁶ et R⁷ représentent ensemble l'oxygène, une oxime ou une O(C₁₋₅-alkyl)-oxime,
R¹⁴ et R¹⁵ peuvent signifier dans chaque cas l'hydrogène ou signifient ensemble un groupe méthylène ou une liaison supplémentaire,
R¹⁶ est l'hydrogène,
R¹⁷ est un groupe CHXY dans lequel X représente un atome d'hydrogène, le fluor,
un groupe hydroxy ou un groupement C₁₋₄-alkyle, et Y représente un atome d'hydrogène ou le fluor, où
quand X est hydroxy, Y ne peut être que l'hydrogène,
R¹⁸ est un atome d'hydrogène ou un groupe méthyle,
où quand R¹⁸ est un groupe méthyle, R¹⁷ peut être un sulfamate SO₃NHR¹,
où dans les cycles B et D du squelette stéroïde, les traits en pointillés peuvent signifier jusqu'à deux doubles liaisons,
ainsi que leurs sels pharmaceutiquement acceptables.

15. Estra-1,3,5(10)-trién-3-yl-sulfamates 2-substitués selon la revendication 14 où R¹ représente l'hydrogène ou C₁₋₅-acyle.

16. Estra-1,3,5(10)-trién-3-yl-sulfamates 2-substitués selon la revendication 14 où R² représente méthyle, éthyle, méthoxy, éthoxy ou 2,2,2-trifluoroéthoxy.

17. Estra-1,3,5(10)-trién-3-yl-sulfamates 2-substitués selon la revendication 14 où, quand R² est un alkyle, R¹⁷ représente C₁₋₅-alcoxy.

18. Estra-1,3,5(10)-trién-3-yl-sulfamates 2-substitués selon la revendication 14 où R⁶ et R⁷ sont dans chaque cas l'hydrogène ou représentent ensemble une oxime.

19. Estra-1,3,5(10)-trién-3-yl-sulfamates 2-substitués selon la revendication 14 où R¹⁴ et R¹⁵ sont dans chaque cas l'hydrogène ou représentent ensemble un groupe méthylène.

20. Estra-1,3,5(10)-trién-3-yl-sulfamates 2-substitués selon la revendication 14 où R¹⁷ représente méthyle, difluorométhyle ou hydroxyméthyle.

21. Estra-1,3,5(10)-trién-3-yl-sulfamates 2-substitués selon la revendication 14 où R¹⁸ représente l'hydrogène ou méthyle.

22. Estra-1,3,5(10)-trién-3-yl-sulfamates 2-substitués selon la revendication 14 où le composé est choisi dans le groupe de composés consistant en
2-éthyl-17β-méthoxy-estra-1,3,5(10)-trién-3-yl-sulfamate,
2-méthoxy-17β-méthyl-estra-1,3,5(10)-trién-3-yl-sulfamate,
17β-difluorométhyl-2-méthoxy-estra-1,3,5(10)-trién-3-yl-sulfamate,
17β-hydroxyméthyl-2-méthoxy-estra-1,3,5(10)-trién-3-yl-sulfamate,
2,17β-diméthoxy-6-oximino-18a-homoestra-1,3,5(10)-trién-3-yl-sulfamate,
17β-éthoxy-2-méthoxy-6-oximino-18a-homoestra-1,3,5(10)-trién-3-yl-sulfamate,

23. Estra-1,3,5(10)-trién-3-yl-sulfamates 2-substitués selon l'une des revendications 14 à 22 pour la production d'un agent pharmaceutique pour le traitement des maladies tumorales des glandes génitales masculines et féminines, des organes sexuels masculins et féminins, y compris des glandes mammaires.

24. Estra-1,3,5(10)-trién-3-yl-sulfamates 2-substitués selon l'une des revendications 14 à 23 pour la production d'un agent pharmaceutique pour le traitement du cancer du sein.

25. Estra-1,3,5(10)-trién-3-yl-sulfamates 2-substitués selon l'une des revendications 14 à 24 pour la production d'un agent pharmaceutique pour le traitement du cancer de la prostate.

26. Estra-1,3,5(10)-trién-3-yl-sulfamates 2-substitués selon la revendication 25 pour la production d'un agent pharmaceutique pour le traitement du cancer de la prostate, où au moins un principe actif supplémentaire est utilisé.
